(19) Europäisches Patentamt
European Patent Office
Office européen des brevets

(11) Veröffentlichungsnummer: **0 264 672**
**A2**

(12) **EUROPÄISCHE PATENTANMELDUNG**

(21) Anmeldenummer: **87114214.7**

(22) Anmeldetag: **29.09.87**

(51) Int. Cl.4: **C07D 251/16**, C07D 251/46, C07D 251/18, C07D 251/22, A01N 47/42

(30) Priorität: **14.10.86 DE 3634929**

(43) Veröffentlichungstag der Anmeldung: **27.04.88 Patentblatt 88/17**

(84) Benannte Vertragsstaaten: **CH DE FR GB LI**

(71) Anmelder: **BAYER AG**
**D-5090 Leverkusen(DE)**

(72) Erfinder: **Fest, Christa, Dr.**
**Im Johannistal 20**
**D-5600 Wuppertal 1(DE)**
Erfinder: **Riebel, Hans-Jochem, Dr.**
**In der Beek 92**
**D-5600 Wuppertal 1(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch-Gladbach 2(DE)**
Erfinder: **Strang, Robert Harry, Dr.**
**Unterdorfstrasse 6A**
**D-4000 Düsseldorf 31(DE)**

(54) **Phenylsulfonylisoharnstoffe.**

(57) Die Erfindung betrifft neue Phenylsulfonylisoharnstoffe der allgemeinen Formel (I)

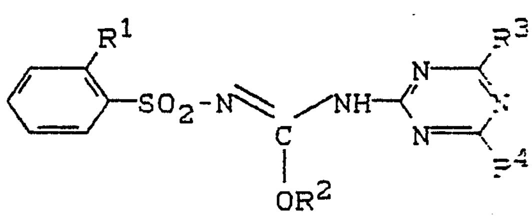

(I)

(worin $R^1$, $R^2$, $R^3$ und $R^4$ die in der Beschreibung angegebenen Bedeutungen haben), ein Verfahren zu ihrer Herstellung und ihre Verwendung als Herbizide.

EP 0 264 672 A2

Xerox Copy Centre

## Phenylsulfonylisoharnstoffe

Die Erfindung betrifft neue Phenylsulfonylisoharnstoffe, ein Verfahren zu ihrer Herstellung, sowie ihre Verwendung als Herbizide.

Es ist bekannt, daß bestimmte Isoharnstoffe, wie z.B. N'-(2-Chlor-benzolsulfonyl)-N''-(4,6-dimethyl-1,3,5-triazin-2-yl)-O-phenyl-isoharnstoff, herbizide Eigenschaften besitzen. Die Wirkung dieser Verbindungen ist jedoch nicht immer ganz befriedigend (vgl. z. B. EP-A 173 957).

Es wurden nun neue Phenylsulfonylisoharnstoffe der allgemeinen Formel (I)

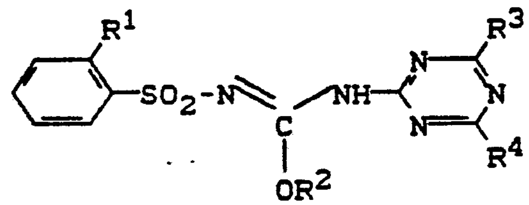

(I)

in welcher

$R^1$ für Halogen [wie insbesondere Fluor, Chlor, Brom und/oder Iod], cyano $C_1$-$C_6$-Alkyl [welches gegebenenfalls durch Fluor, Chlor, Brom, Cyano, $C_1$-$C_4$-Alkoxy, $C_3$-$C_6$-Cycloalkyl oder Phenyl substituiert ist] oder für $C_1$-$C_4$-Alkoxy [welches gegebenenfalls durch Chlor, Brom, Cyano oder $C_1$-$C_4$-Alkoxy substituiert ist] steht,

$R^2$ für gegebenenfalls substituiertes Aryl steht,

$R^3$ für Wasserstoff, Halogen, Hydroxy, $C_1$-$C_6$-Alkylamino, Di-($C_1$-$C_6$-alkyl)-amino oder für gegebenenfalls substituiertes $C_1$-$C_6$-Alkyl, $C_1$-$C_6$-Alkoxy und $C_1$-$C_6$-Alkylthio steht und

$R^4$ für Wasserstoff, Halogen, Hydroxy, für gegebenenfalls substituiertes $C_1$-$C_6$-Alkyl, $C_1$-$C_6$-Alkoxy und $C_1$-$C_6$-Alkylthio, für $C_1$-$C_6$-Alkylamino oder Di-($C_1$-$C_6$-alkyl)-amino steht,

gefunden, wobei jedoch die folgende Verbindung ausgenommen wird,

N'-(2-Chlor-benzolsulfonyl)-N''-(4,6-dimethyl-1,3,5-triazin-2-yl)-O-phenyl-isoharnstoff.

Man erhält die neuen Phenylsulfonylisoharnstoffe der allgemeinen Formel (I), wenn man Iminokohlensäureester der Formel (II)

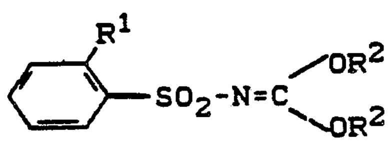

(II)

in welcher

$R^1$ und $R^2$ die oben angegebenen Bedeutungen haben,

mit 2-Amino-triazinen der Formel (III)

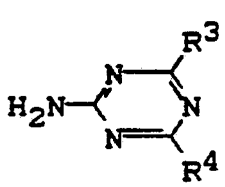

(III)

in welcher

$R^3$ und $R^4$ die oben angegebenen Bedeutungen haben,

in Gegenwart von Säureakzeptoren und gegebenenfalls in Gegenwart von Verdünnungsmitteln umsetzt.

Die neuen Phenylsulfonylisoharnstoffe der Formel (I) zeichnen sich durch eine starke herbizide Wirksamkeit aus. Überraschenderweise zeigen die neuen Verbindungen der Formel (I) eine erheblich bessere herbizide Wirkung als vorbekannte Isoharnstoffe gleicher Wirkungsrichtung.

Gegenstand der Erfindung sind vorzugsweise Verbindungen der Formel (I), in welcher

$R^1$ für Fluor, Chlor, Brom, Cyano, $C_1$-$C_4$-Alkyl [welches gegebenenfalls duch Fluor, Chlor, Brom, Cyano, $C_1$-$C_2$-Alkoxy oder Phenyl substituiert ist] oder für $C_1$-$C_2$-Alkoxy [welches gegebenenfalls durch Chlor, Brom, Cyano oder $C_1$-$C_2$-Alkoxy substituiert ist] steht,

$R^2$ für einen Phenylrest steht, welcher gegebenenfalls substituiert ist durch einen oder mehrere Reste aus der Reihe

Halogen [wie insbesondere Fluor, Chlor, Brom und Iod], Cyano, Nitro, Hydroxy, Carboxy, $C_1$-$C_6$-Alkyl [welches gegebenenfalls durch Fluor, Chlor, Brom, Nitro, Cyano, Hydroxy, Carboxy, $C_1$-$C_4$-Alkoxy-carbonyl, $C_1$-$C_4$-Alkoxy, $C_1$-$C_4$-Alkylthio oder Phenyl substituiert ist], $C_3$-$C_6$-Cycloalkyl, $C_1$-$C_4$-Alkoxy [welches gegebenenfalls durch Fluor, Chlor, Brom, Cyano, Carboxy, $C_1$-$C_4$-Alkoxy, $C_1$-$C_4$-Alkylthio oder $C_1$-$C_4$-Alkoxy-carbonyl substituiert ist], $C_1$-$C_4$-Alkylthio [welches gegebenenfalls durch Fluor, Chlor, Brom, Cyano, Carboxy, $C_1$-$C_4$-Alkoxy-carbonyl substituiert ist], Amino, $C_1$-$C_4$-Alkyl-amino bzw. Di-($C_1$-$C_4$-alkyl)-amino [welche gegebenenfalls durch Fluor, Chlor, Brom, Cyano, Carboxy, $C_1$-$C_4$-Alkoxy oder $C_1$-$C_4$-Akoxy-carbonyl substituiert sind], $C_1$-$C_4$-Alkyl-carbonyl-amino, $C_1$-$C_4$-Alkoxy-carbonylamino, (Di)-$C_1$-$C_4$-Alkylamino-carbonyl-amino, Formyl, $C_1$-$C_4$-Alkyl-carbonyl, Benzoyl, $C_1$-$C_4$-Alkoxy-carbonyl, Phen oxy-carbonyl, Benzyloxycarbonyl, Phenyl [welches gegebenenfalls durch Fluor, Chlor, Brom, Cyano, Nitro, Hydroxy oder Methyl substituiert ist], Phenoxy, Phenylthio, Phenylsulfonyl, Phenylamino oder Phenylazo [welche gegebenenfalls durch Fluor, Chlor, Brom, Cyano, Nitro, Methyl und/oder Trifluormethyl substituiert sind], Pyridoxy oder Pyrimidoxy [welche gegebenenfalls durch Fluor, Chlor, Brom, Cyano, Nitro, Methyl und/oder Trifluormethyl substituiert sind], $C_1$-$C_4$-Alkyl-carbonyloxy, $C_1$-$C_4$-Alkoxy-carbonyloxy, $C_1$-$C_4$-Alkyl-amino-carbonyloxy und Di-($C_1$-$C_4$-alkyl)-amino-carbonyloxy, oder welcher gegebenenfalls durch eine Alkylenkette [welche gegebenenfalls verzweigt und/oder durch ein oder mehrere Sauerstoffatome unterbrochen ist] oder einen Benzorest [welcher gegebenenfalls durch Fluor, Chlor, Brom, Cyano, Nitro, Methyl und/oder Trifluormethyl substituiert ist] anelliert ist; worin weiter

$R^3$ für Wasserstoff, Fluor, Chlor, Brom, Hydroxy, $C_1$-$C_4$-Alkylamino, Di-($C_1$-$C_4$-alkyl)-amino, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio [welches gegebenenfalls durch Fluor und/oder Chlor substituiert ist] steht und

$R^4$ für Wasserstoff, Fluor, Chlor, Brom, Hydroxy, $C_1$-$C_4$-Alkyl [welches gegebenenfalls durch Fluor und/oder Chlor substituirt ist], $C_1$-$C_4$-Alkoxy [welches gegebenenfalls durch Fluor und/oder Chlor substituiert ist], $C_1$-$C_4$-Alkylthio [welches gegebenenfalls durch Fluor und/oder Chlor substituiert ist], $C_1$-$C_4$-Alkylamino oder Di-($C_1$-$C_4$-alkyl)-amino steht,

wobei jedoch die folgende Verbindung ausgenommen wird: N'-(2-Chlor-benzolsulfonyl)-N''-(4,6-dimethyl-1,3,5-triazin-2-yl)-O-phenyl-isoharnstoff.

Besonders bevorzugt sind diejenigen Verbindungen der Formel (I), in welcher

$R^1$ für Fluor, Chlor, Brom, Cyano, Methyl, Ethyl, Trifluormethyl, Methoxy, 2-Chlorethoxy oder Benzyl steht,

$R^2$ für einen Phenylrest steht, welcher gegebenenfalls substituiert ist durch einen oder zwei Reste aus der Reihe

Fluor, Chlor, Brom, Iod, Cyano, Nitro, Hydroxy, Carboxy, $C_1$-$C_3$-Alkoxy-carbonyl, $C_1$-$C_4$-Alkyl, Trifluormethyl, Hydroxymethyl, Methoxycarbonylmethyl, Phenyl-$C_1$-$C_3$-alkyl, Cyclohexyl, $C_1$-$C_3$-Alkoxy, Trifluormethoxy, $C_1$-$C_3$-Alkylthio, Trifluormethylthio, Dimethylamino, Amino, Acetylamino, Methylaminocarbonyl, Formyl, Acetyl, Benzoyl, Phenyl, Hydroxyphenyl, Phenoxy [welches gegebenenfalls durch Chlor und/oder Trifluormethyl substituiert ist], Phenylamino, Phenylazo, Pyridoxy [welches gegebenenfalls durch Chlor und/oder Trifluormethyl substituiert ist], oder welcher gegebenenfalls benzanelliert ist; worin weiter

$R^3$ für Fluor, Chlor, Methyl, Ethyl, Methoxy, Ethoxy, Methylthio, Ethylthio, Methylamino, Ethylamino, Dimethylamino oder Diethylamino steht und

$R^4$ für Fluor, Chlor, Methyl, Ethyl, Methoxy, Ethoxy, Methylthio, Ethylthio, Methylamino, Ethylamino, Dimethylamino oder Diethylamino steht,

wobei jedoch die folgende Verbindung ausgenommen wird: N'-(2-Chlor-benzolsulfonyl)-N''-(4,6-dimethyl-1,3,5-triazin-2-yl)-O-phenyl-isoharnstoff.

Verwendet man beispielsweise für das erfindungsgemäße Verfahren N-(2-Chlor-benzolsulfonyl)-iminokohlensäure-O,O-diphenylester und 2-Amino-4-methoxy-6-methyl-1,3,5-triazin als Ausgangsstoffe, so kann der Reaktionsablauf durch das folgende Formelschema wiedergegeben werden:

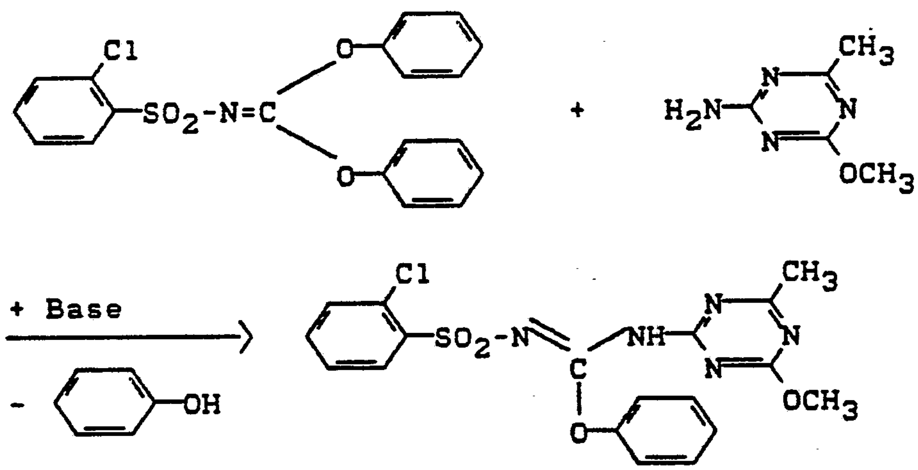


Die beim erfindungsgemäßen Verfahren als Ausgangsstoffe zu verwendenden Iminokohlensäureester sind durch die Formel (II) allgemein definiert. In dieser Formel (II) stehen $R^1$ und $R^2$ vorzugsweise für diejenigen Reste, die bereits im Zusammenhang mit der Beschreibung der erfindungsgemäßen Stoffe der Formel (I) vorzugsweise für diese Substituenten genannt wurden.

Die Verbindungen der Formel (II) sind neu und Gegenstand einer eigenen, hiermit gleichzeitig eingereichten und nicht zum vorveröffentlichten Stand der Technik gehörenden Patentanmeldung (vgl. die deutsche Anmeldung P 36 34 926.7).

Man erhält die Verbindungen der Formel (II), wenn man Iminokohlensäuredichloride der Formel (IV)

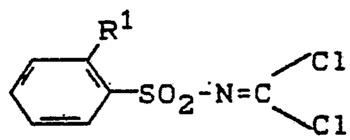 (IV)

in welcher
$R^1$ die oben angegebenen Bedeutungen hat,
mit Verbindungen der Formel (V)

$R^2$-OM (V)

in welcher
$R^2$ die oben angegebenen Bedeutungen hat und
M für Wasserstoff oder ein Alkalimetallatom steht,
gegebenenfalls in Gegenwart von Säureakzeptoren, wie z. B. Natrium-oder Kaliumhydroxid, und gegebenenfalls in Gegenwart von inerten Verdünnungsmitteln, wie z.B. Aceton, Methylethylketon oder Acetonitril, bei Temperaturen zwischen 10°C und 100°C umsetzt.

Die Iminokohlensäuredichloride der Formel (IV) sind zum Teil bekannt und/oder lassen sich nach bekannten Verfahren herstellen (vergl. z. B. Chem. Ber. 99, 2900 (1966), CA 90:137826b; Angew. Chem. 77, 430 (1965)). Neu und ebenfalls Gegenstand einer eigenen, hiermit gleichzeitig eingereichten und nicht zum vorveröffentlichten Stand der Technik gehörenden Patentanmeldung (vgl. die deutsche Anmeldung P 36 34 926.7) sind die Iminokohlensäuredichloride der Formel (IVa)

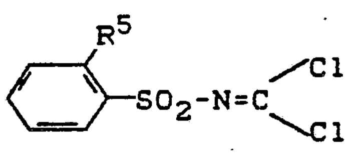 (IVa)

in welcher
$R^5$ für Halogen [wie insbesondere Fluor, Chlor, Brom und/oder Iod], Cyano, $C_2$-$C_6$-Alkyl, $C_1$-$C_6$-Alkyl [welches durch Fluor, Chlor, Brom, Cyano, $C_1$-$C_4$-Alkoxy, $C_3$-$C_6$-Cycloalkyl oder Phenyl substituiert ist] oder für $C_1$-$C_4$-Alkoxy [welches gegebenenfalls durch Chlor, Brom, Cyano oder $C_1$-$C_4$-Alkoxy substituiert ist] steht.

Man erhält die neuen Verbindungen der Formel (IVa), wenn man Iminodithiokohlensäuredimethylester der Formel (VI)

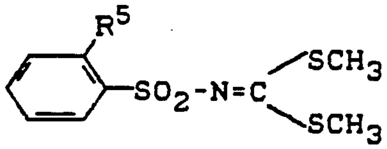

(VI)

in welcher
$R^5$ die oben bei Formel (IVa) angegebenen Bedeutungen hat,
in Gegenwart von inerten Verdünnungsmitteln, wie z. B. Tetrachlorkohlenstoff, bei Temperaturen zwischen 0 °C und 25 °C mit Chlorierungsmitteln, wie z. B. Sulfurylchlorid oder Chlor, umsetzt.

Die Iminodithiokohlensäuredimethylester der Formel (VI) sind bekannt und/oder lassen sich nach bekannten Methoden herstellen (vergl. z. B. EP-A 121 082, EP-A 151 554 und EP-A 173 957).

Die weiterhin als Ausgangsstoffe für die Herstellung der Verbindungen der Formel (II) einzusetzenden Verbindungen der Formel (V) sind allgemein bekannte Verbindungen der organischen Chemie.

Als Beispiele für die Iminokohlensäureester der Formel (II) seien genannt:

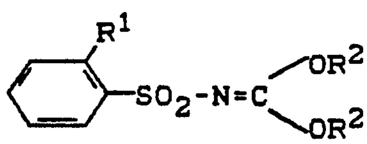

(II)

Tabelle 1

| 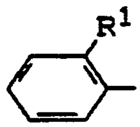 | $R^2$ |
|---|---|
| 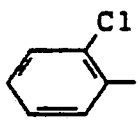 | 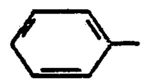 |
| 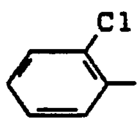 | 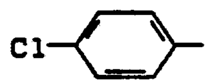 |
| 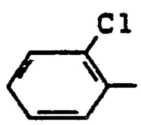 | 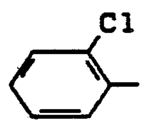 |
| 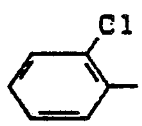 | 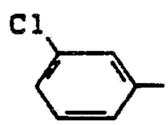 |
| 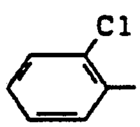 | 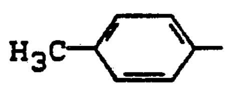 |
| 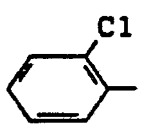 | 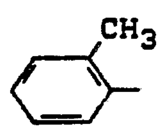 |
| 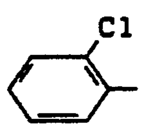 | 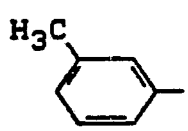 |
| 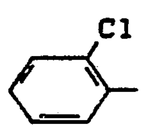 | 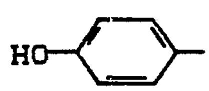 |

Tabelle 1 - Fortsetzung

| 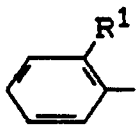 | $R^2$ |
| --- | --- |
| 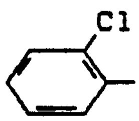 | 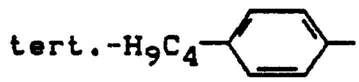 |
| 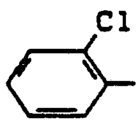 | 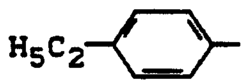 |
| 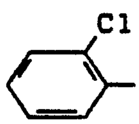 | 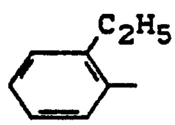 |
| 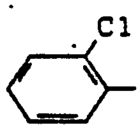 | 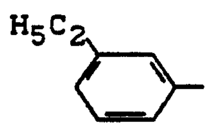 |
| 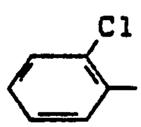 | 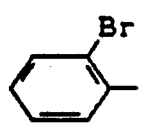 |
| 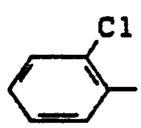 | 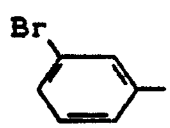 |
| 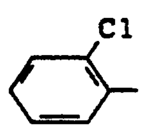 | 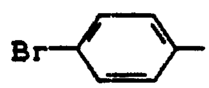 |
| 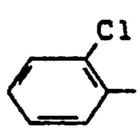 | 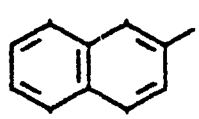 |

<u>Tabelle 1</u> - Fortsetzung

| 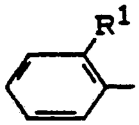 | $R^2$ |
|---|---|
| 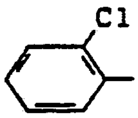 | 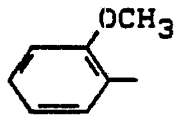 |
| 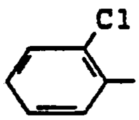 | 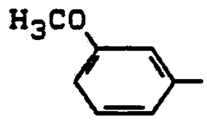 |
| 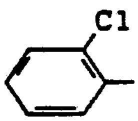 | 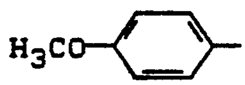 |
| 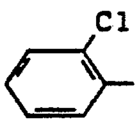 | 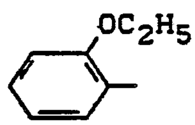 |
| 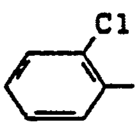 | 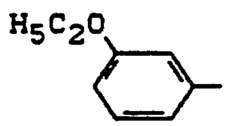 |
| 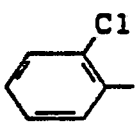 | 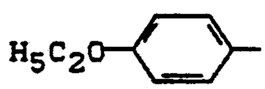 |
| 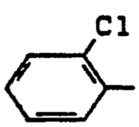 | 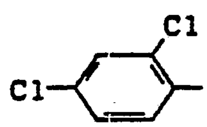 |
| 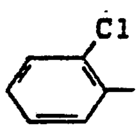 | 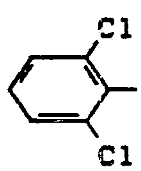 |

<u>Tabelle 1</u> - Fortsetzung

| 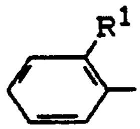 | $R^2$ |
|---|---|
| 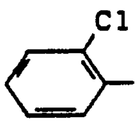 | 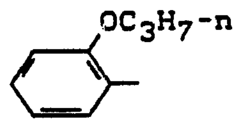 |
| 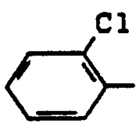 | 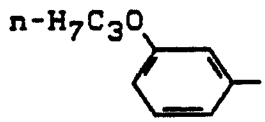 |
| 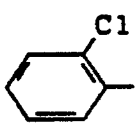 | 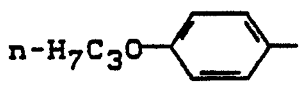 |
| 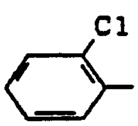 | 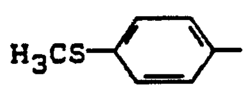 |
| 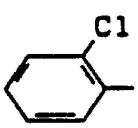 | 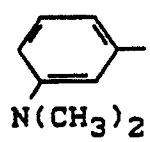 |
| 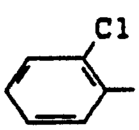 | 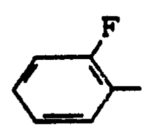 |
| 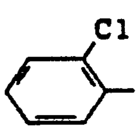 | 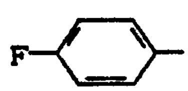 |
| 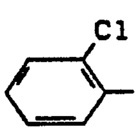 | 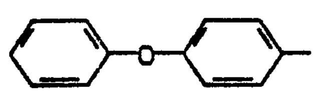 |

<u>Tabelle 1</u> - Fortsetzung

| 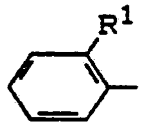 | $R^2$ |
|---|---|
| 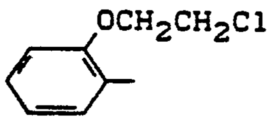 | 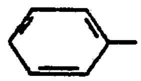 |
| 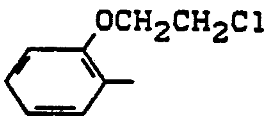 | 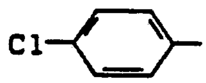 |
| 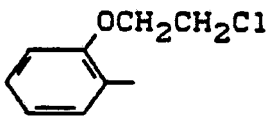 | 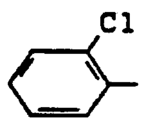 |
| 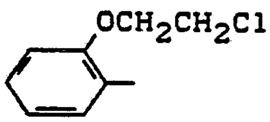 | 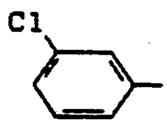 |
| 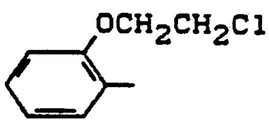 | 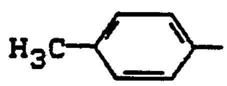 |
| 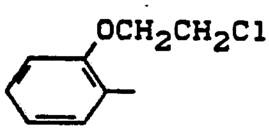 | 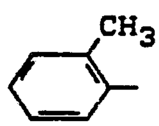 |
| 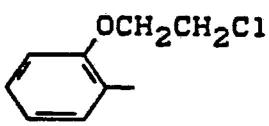 | 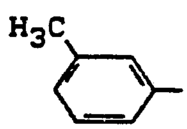 |
| 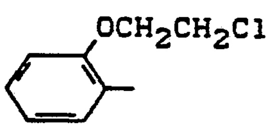 | 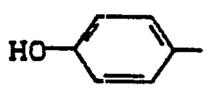 |

Tabelle 1 - Fortsetzung

| 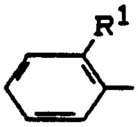 | $R^2$ |
|---|---|
| 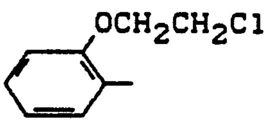 | 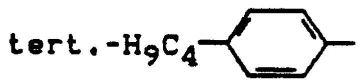 |
| 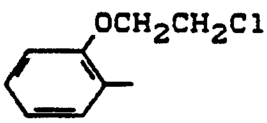 | 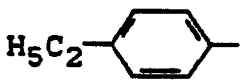 |
| 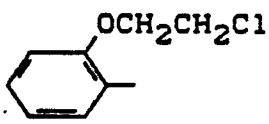 | 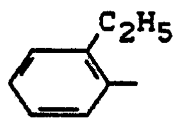 |
| 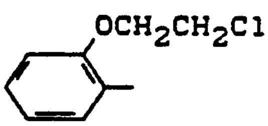 | 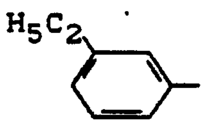 |
| 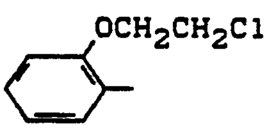 | 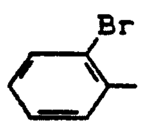 |
| 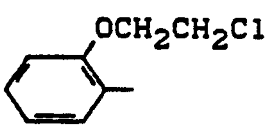 | 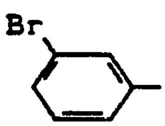 |
| 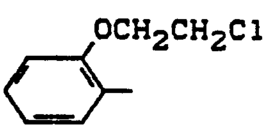 | 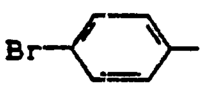 |
| 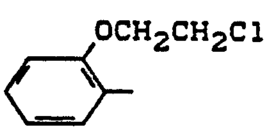 | 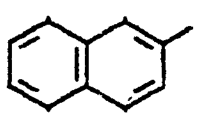 |

Tabelle 1 - Fortsetzung

| 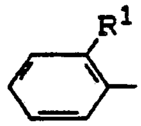 | $R^2$ |
| --- | --- |
| 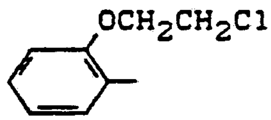 | 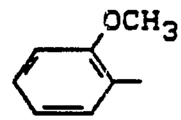 |
| 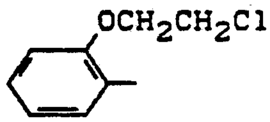 | 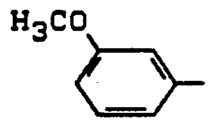 |
| 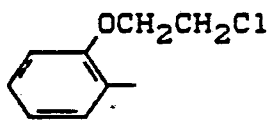 | 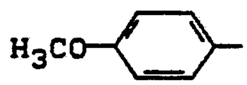 |
| 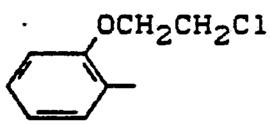 | 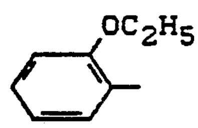 |
| 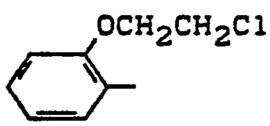 | 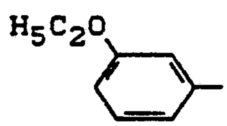 |
| 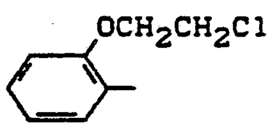 | 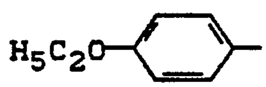 |
| 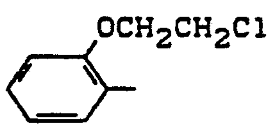 | 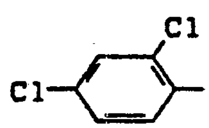 |
| 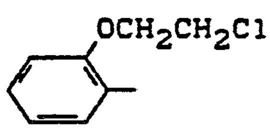 | 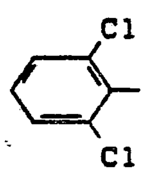 |

Tabelle 1 - Fortsetzung

| 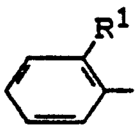 | $R^2$ |
|---|---|
| 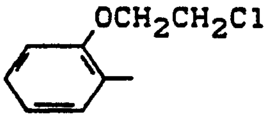 | 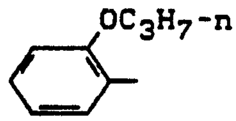 |
| 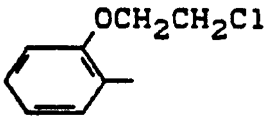 | 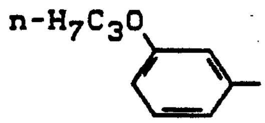 |
| 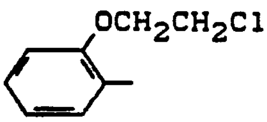 | 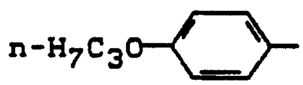 |
| 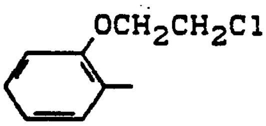 | 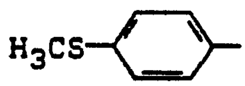 |
| 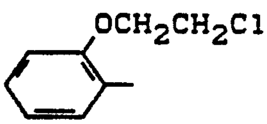 | 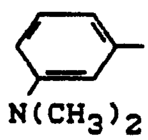 |
| 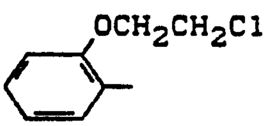 | 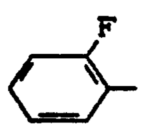 |
| 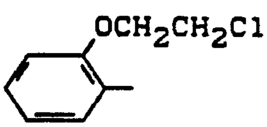 | 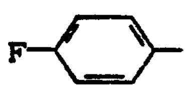 |
| 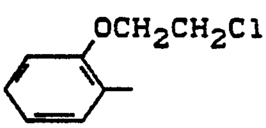 | 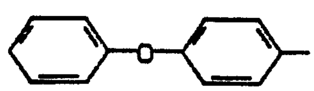 |

<u>Tabelle 1</u> - Fortsetzung

| 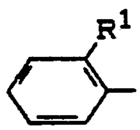 | $R^2$ |
| --- | --- |
| 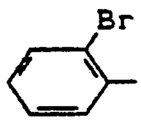 | 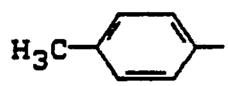 |
| 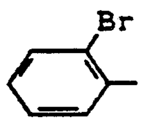 | 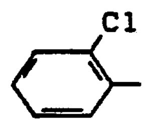 |
| 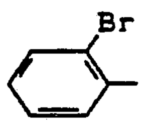 | 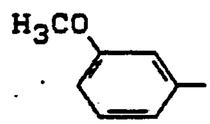 |
| 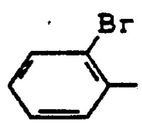 | 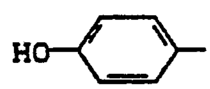 |
| 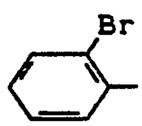 | 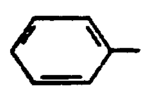 |
| 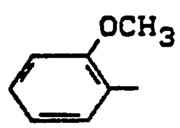 | 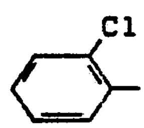 |
| 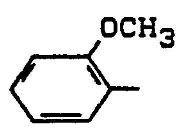 | 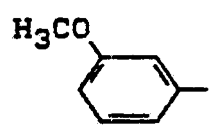 |

Tabelle 1 - Fortsetzung

| 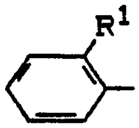 | $R^2$ |
| --- | --- |
| 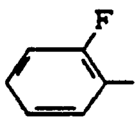 | 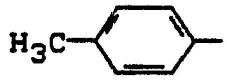 |
| 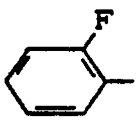 | 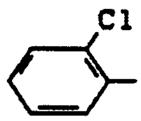 |
| 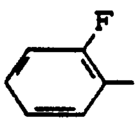 | 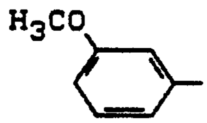 |
| 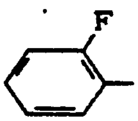 | 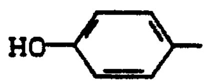 |
| 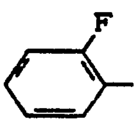 | 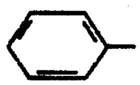 |
| 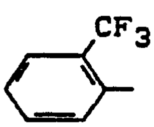 | 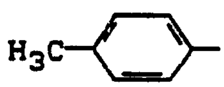 |
| 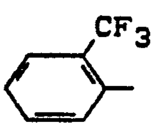 | 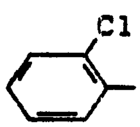 |
| 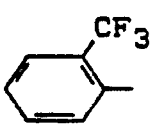 | 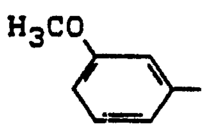 |

Tabelle 1 - Fortsetzung

| 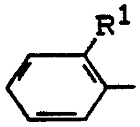 | $R^2$ |
| --- | --- |
| 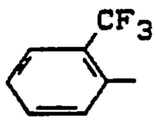 | 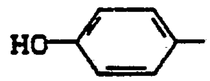 |
| 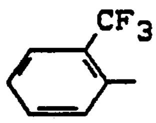 | 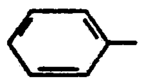 |
| 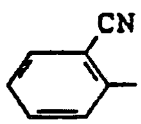 | 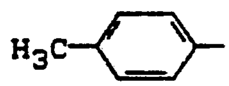 |
| 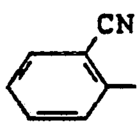 | 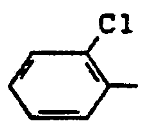 |
| 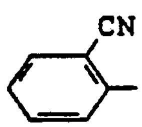 | 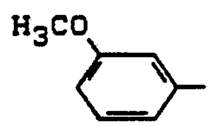 |
| 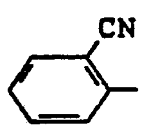 | 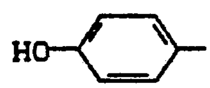 |
| 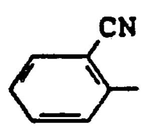 | 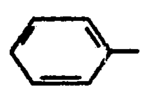 |
| 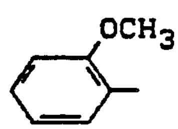 | 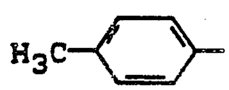 |

Tabelle 1 - Fortsetzung

| 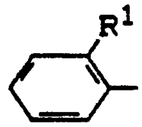 | $R^2$ |
|---|---|
| 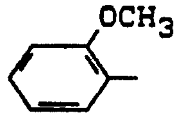 | 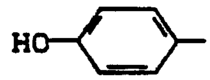 |
| 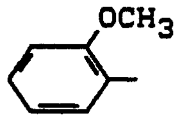 | 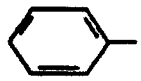 |
| 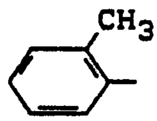 | 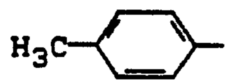 |
| 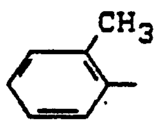 | 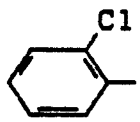 |
| 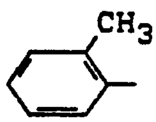 | 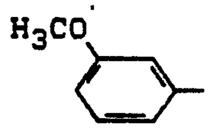 |
| 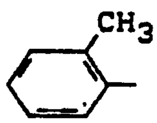 | 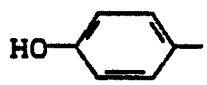 |
| 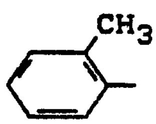 | 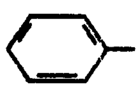 |

Die beim erfindungsgemäßen Verfahren außerdem als Ausgangsstoffe zu verwendenden 2-Amino-triazine sind durch die Formel (III) allgemein definiert. In dieser Formel (III) stehen $R^3$ und $R^4$ vorzugsweise für diejenigen Reste, die bereits im Zusammenhang mit der Beschreibung der erfindungsgemäßen Stoffe der Formel (I) vorzugsweise für diese Substituenten genannt wurden.

Als Beispiele für die 2-Amino-triazine der Formel (III) seien genannt:

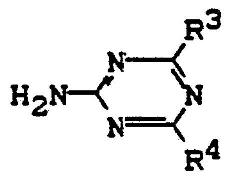 (III)

Tabelle 2

| $R^3$ | $R^4$ |
|---|---|
| $CH_3$ | $CH_3$ |
| $OCH_3$ | $OCH_3$ |
| $CH_3$ | $OCH_3$ |
| $C_2H_5$ | $C_2H_5$ |
| $OC_2H_5$ | $OC_2H_5$ |
| $OC_2H_5$ | $CH_3$ |
| $OC_2H_5$ | $OCH_3$ |
| $CH_3$ | $SCH_3$ |
| $CH_3$ | $SC_2H_5$ |
| $OCH_3$ | $SCH_3$ |
| $OC_2H_5$ | $SCH_3$ |

Tabelle 2 - Fortsetzung

| $R^3$ | $R^4$ |
|---|---|
| $OCH_3$ | $SC_2H_5$ |
| $OC_2H_5$ | $SC_2H_5$ |
| $CH_3$ | $NHCH_3$ |
| $CH_3$ | $N(CH_3)_2$ |
| $CH_3$ | $NHC_2H_5$ |
| $CH_3$ | $N(C_2H_5)_2$ |
| $OCH_3$ | $NHCH_3$ |
| $OCH_3$ | $NHC_2H_5$ |
| $OCH_3$ | $N(CH_3)_2$ |
| $OCH_3$ | $N(C_2H_5)_2$ |
| $OC_2H_5$ | $N(C_2H_5)_2$ |
| $OC_2H_5$ | $N(CH_3)_2$ |
| $SCH_3$ | $NHCH_3$ |
| $SCH_3$ | $N(CH_3)_2$ |
| $SCH_3$ | $NHC_2H_5$ |
| $SCH_3$ | $N(C_2H_5)_2$ |
| $SC_2H_5$ | $NHCH_3$ |
| $SC_2H_5$ | $N(CH_3)_2$ |
| $OC_2H_5$ | $NHCH_3$ |
| $OC_2H_5$ | $NHC_2H_5$ |
| $SC_2H_5$ | $N(C_2H_5)_2$ |
| Cl | $CH_3$ |
| Cl | $OCH_3$ |

Die 2-Aminotriazine der Formel (III) sind bekannt und/oder können nach an sich bekannten Verfahren hergestellt werden.

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Methyl-isopropyl-und Methyl-isobutyl-keton, Ester wie Essigsäuremethylester und -ethylester, Nitrile wie z. B. Acetonitrilund Propionitril, Amide wie z. B. Dimethylformamid, Dimethylacetamid und N-methyl-pyrrolidon sowie Dimethylsulfoxid, Tetramethylensulfon und Hexamethylphosphorsauretriamid.

Als Säureakzeptoren werden bei dem erfindungsgemäßen Verfahren zur Herstellung der neuen Verbindungen der Formel (I) alle üblicherweise für derartige Umsetzungen verwendbaren Säurebindemittel eingesetzt. Vorzugsweise in Frage kommen Alkalimetallhydroxide wie z. B. Natrium-und Kaliumhydroxid, Alkalimetallhydride wie z. B. Natriumhydrid, Erdalkalihydroxide wie z. B. Calciumhydroxid, Alkalicarbonate und -alkoholate wie Natrium-und Kaliumcarbonat, Natrium-und Kaliummethylat bzw. -ethylat oder Kalium-tert.-butylat.

Die Reaktionen werden im allgemeinen bei Temperaturen zwischen -20 °C und 150 °C, vorzugsweise bei Temperaturen zwischen 0 °C und 80 °C durchgeführt. Die Reaktionen werden in allgemeinen bei Normaldruck durchgeführt.

Zur Durchführung des erfindungsgemäßen Verfahrens werden die Ausgangsstoffe der Formeln (II) und (III) und gegebenenfalls der geeignete Säureakzeptor in äquimolaren Mengen eingesetzt.

Bevorzugt werden eine Verbindung der Formel (III), der Säureakzeptor und das Verdünnungsmittel wie z. B. Tetrahydrofuran vorgelegt und einige Stunden gerührt. Anschliessend wird die Verbindung der Formel (II) zugegeben. Nach der Umsetzung wird die Reaktionslösung eingeengt, mit Wasser versetzt, über Kieselgel abgesaugt und mit einer Mineralsäure wie z. B. Salzsäure schwach angesäuert. Die Lösung wird in einem organischen Verdünnungsmittel wie z. B. Methylenchlorid aufgenommen. Die organische Phase wird mit gesättigter Natriumcarbonatlösung und anschließend mit Wasser gewaschen, getrocknet und eingeengt. Die Reinigung der Rohprodukte geschieht nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Die erfindungsgemäßen Verbindungen der Formel (I) eignen sich zur selektiven Bekämpfung dikotyler Unkräuter in monokotylen Kulturen wie z. B. Gerste und Weizen im Vor-und im Nachauflaufverfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festern Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinemehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen können bekannte Herbizide verwendet werden wie z. B. N-(2-Benzthiazolyl)-N,N'-dimethylharnstoff, 3-(3-Chlor-4-methylphenyl)-1,1-dimethylharnstoff, 3-(4-Isopropylphenyl)-1,1-dimethylharnstoff, 2-Chlor-N-{[(4-methoxy-6-methyl-1,3,5-triazin-2-yl)-amino]-carbonyl}-benzolsulfonamid, 2-Ethylamino-6-(1,1-dimethylethyl-amino)-4-methylthio-1,3,5-triazin, 3-(2,4-Dichlorphenoxy)-6-nitro-benzoesäuremethylester, 2-[4-(2,4-Dichlorphenoxy)-phenoxy]-propionsäuremethylester, das R-Enantiomere des 2-{4-[(3,5-Dichlor-2-pyridinyl)-oxy]-phenoxy}-propionsäure-(trimethylsilyl)-methylesters, 2,4-Dichlorphenoxyessigsäure, 2-(2,4-Dichlorphenoxy)-propionsäure, 4-Chlor-2-methyl-phenoxy-essigsäure, 2-(4-Chlor-2-methyl-phenoxy)-propionsäure, 3,5-Diiod-4-hydroxy-benzonitril, 3,5-Dibrom-4-hydroxy-benzonitril, N-(1-Ethylpropyl)-3,4-dimethyl-2,6-dinitroanilin, N,N-Diisopropyl-2,3,3-trichlorallylthiolcarbamat, 2-(2-Benzthiazolyloxy)-N-methyl-N-phenylacetamid, 2-[4,5-Dihydro-4-methyl-4-(1-methylethyl)-5-oxo-1H-imidazol-2-yl]-4(5)-methyl-benzoesäuremethylester, [(4-Amino-3,5-dichlor-6-fluor-2-pyridyl)-oxy]-essigsäure und 3-Isopropyl-2,1,3-benzothiadiazinon-(4)-2,2-dioxid. Einige Mischungen besitzen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in eienm größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Die erfindungsgemäßen Wirkstoffe besitzen zum Teil auch eine fungizide Wirksamkeit, insbesondere gegen Pyricularia oryzae am Reis.

Herstellungsbeispiele

Beispiel 1

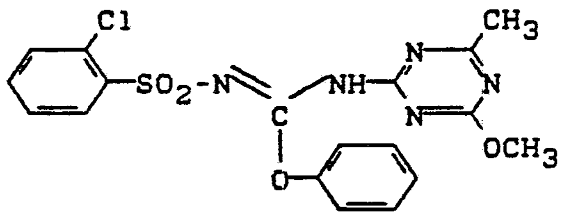

7 g (0,05 Mol) 2-Amino-4-methoxy-6-methyl-1,3,5-triazin werden in 100 ml Tetrahydrofuran gelöst und mit 5,9 g (0,05 Mol) Kalium-tert.-butylat bei 20 °C versetzt. Nach 2 Stunden werden bei 20 °C 19,4 g (0,05 Mol) N-(2-Chlorbenzolsulfonyl)-iminokohlensäure-O,O-diphenylester zugegeben; dann wird 16 Stunden bei 20 °C nachgerührt. Das Reaktionsgemisch wird anschließend auf 500 ml Wasser gegossen und abfiltriert. Das Filtrat wird mit 2 N-Salzsäure schwach angesäuert und mit Methylenchlorid extrahiert. Die organische Phase wird neutral gewaschen, getrocknet und eingeengt. Der Rückstand wird mit einem Gemisch aus Isopropanol/Diisopropylether verrührt, abgesaugt, gewaschen und getrocknet.

Man erhält 15,5 g (71,8 % der Theorie) N'-(2-Chlor-benzolsulfonyl)-N''-(4-methoxy-6-methyl-1,3,5-triazin-2-yl)-O-phenyl-isoharnstoff vom Schmelzpunkt 95 °C.

Beispiel 2

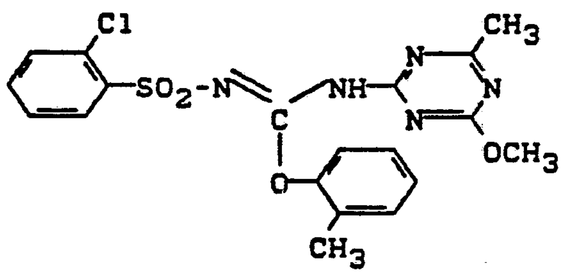

Eine Mischung von 5,6 g (0,04 Mol) 2-Amino-4-methoxy-6-methyl-1,3,5-triazin, 2,7 g (0,08 Mol) Natriumhydrid in Paraffin und 100 ml Tetrahydrofuran wird 15 Stunden bei 25 °C gerührt. Anschließend wird diese Mischung portionsweise mit 16,6 g (0,04 Mol) 2-Chlorbenzolsulfonylimino-kohlensäure-O,O-di-(4-methyl-phenyl)-ester in der Weise versetzt, daß eine Temperatur von 30 °C nicht überschritten wird; anschließend wird 3 Stunden bei 25 °C nachgerührt. Das Reaktionsgemisch wird unter Eiskühlung mit 300 ml Wasser versetzt und die wäßrige Phase wird mit verdünnter Salzsäure auf pH ca. 5 eingestellt. Die wäßrige

Phase wird zweimal mit jeweils 100 ml Methylenchlorid extrahiert, die vereinigten Methylenchloridphasen werden mit Wasser gewaschen und nach dem Trocknen über Natriumsulfat eingeengt. Nach dem Andestillieren (Badtemperatur/Druck : 140 °C/202 Pa) wird der Rückstand mit Ethanol verrieben, abfiltriert und getrocknet.

Man erhält 5,1 g ( 29,5 % der Theorie) N'-(2-Chlorbenzolsulfonyl)-N"-(4-methoxy-6-methyl-1,3,5-triazin-2-yl)-O-(2-methyl-phenyl)-isoharnstoff in Form farbloser Kristalle vom Schmelzpunkt 118 °C.

Analog Beispiel 1 und 2 können die nachfolgenden Verbindungen der Formel (I) hergestellt werden:

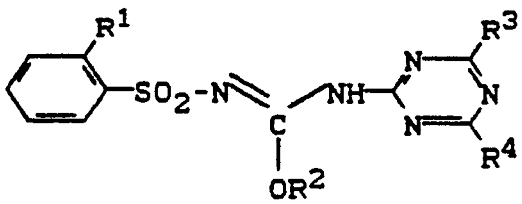

(I)

Tabelle 3

| Beisp.-Nr. | 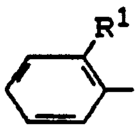 | $R^2$ | $R^3$ | $R^4$ | Fp /[°C] |
|---|---|---|---|---|---|
| 3 | 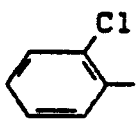 | 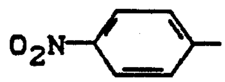 | $OCH_3$ | $OCH_3$ | 125 |
| 4 | 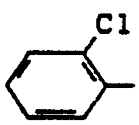 | 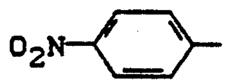 | $CH_3$ | $OCH_3$ | 165 |
| 5 | 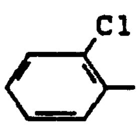 | 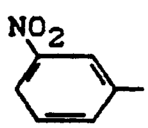 | $CH_3$ | $OCH_3$ | 152 |
| 6 | 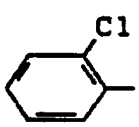 | 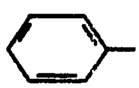 | $CH_3$ | $N(CH_3)_2$ | 128 |
| 7 | 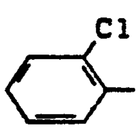 | 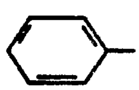 | $CH_3$ | $SCH_3$ | 135 |

Tabelle 3 - Fortsetzung

| Beisp.-Nr. | 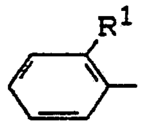 | $R^2$ | $R^3$ | $R^4$ | Fp /[°C] |
|---|---|---|---|---|---|
| 8 | 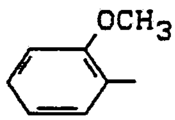 | 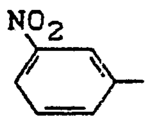 | $OCH_3$ | $OCH_3$ | 175 |
| 9 | 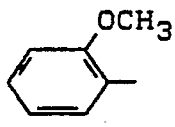 | 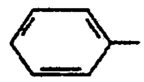 | $OCH_3$ | $OCH_3$ | 176 |
| 10 | 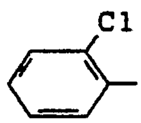 | 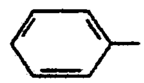 | $OCH_3$ | $OCH_3$ | 96 |
| 11 | 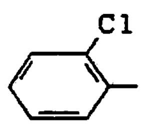 | 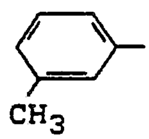 | $CH_3$ | $OCH_3$ | 141 |
| 12 | 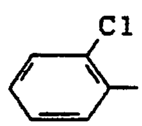 | 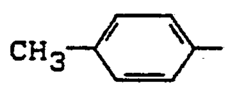 | $CH_3$ | $OCH_3$ | 114 |
| 13 | 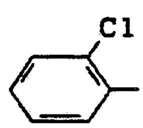 | 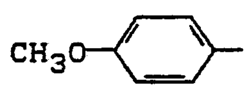 | $CH_3$ | $OCH_3$ | |
| 14 | 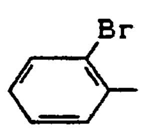 | 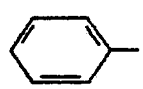 | $CH_3$ | $OCH_3$ | 144 |
| 15 | 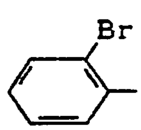 | 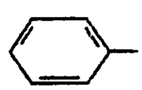 | $OCH_3$ | $OCH_3$ | |

Tabelle 3 - Fortsetzung

| Beisp.-Nr. | 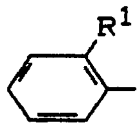 | $R^2$ | $R^3$ | $R^4$ | Fp /[° C] |
|---|---|---|---|---|---|
| 16 | 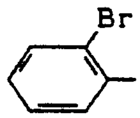 | 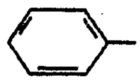 | $CH_3$ | $CH_3$ | |
| 17 | 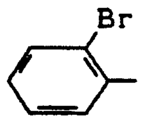 | 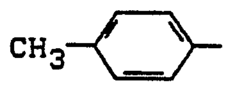 | $CH_3$ | $OCH_3$ | 152 |
| 18 | 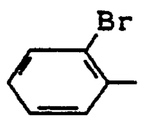 | 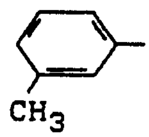 | $CH_3$ | $OCH_3$ | 158 |
| 19 | 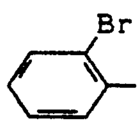 | 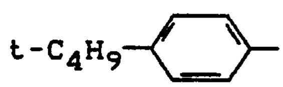 | $CH_3$ | $CH_3$ | |
| 20 | 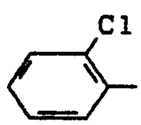 | 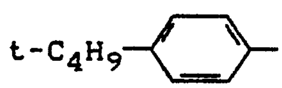 | $CH_3$ | $OCH_3$ | |
| 21 | 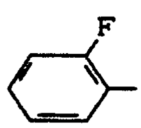 | 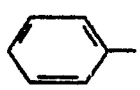 | $CH_3$ | $OCH_3$ | |
| 22 | 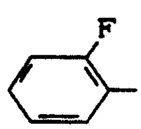 | 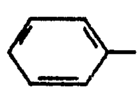 | $OCH_3$ | $OCH_3$ | |
| 23 | 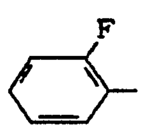 | 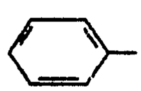 | $CH_3$ | $CH_3$ | |

Tabelle 3 - Fortsetzung

| Beisp.-Nr. | 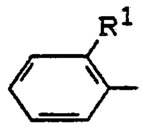 | $R^2$ | $R^3$ | $R^4$ | Fp /[°C] |
|---|---|---|---|---|---|
| 24 | 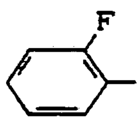 | 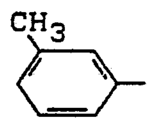 | $CH_3$ | $OCH_3$ | |
| 25 | 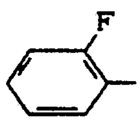 | 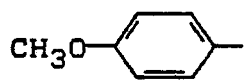 | $CH_3$ | $OCH_3$ | 107 |
| 26 | 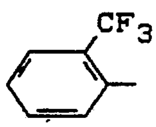 | 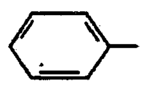 | $CH_3$ | $OCH_3$ | |
| 27 | 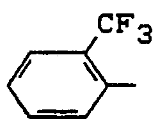 | 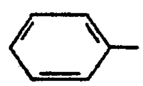 | $OCH_3$ | $OCH_3$ | 103 |
| 28 | 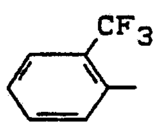 | 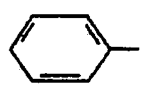 | $CH_3$ | $CH_3$ | |
| 29 | 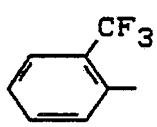 | 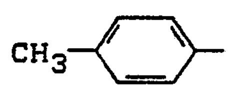 | $CH_3$ | $OCH_3$ | |
| 30 | 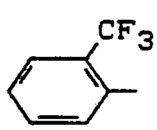 | 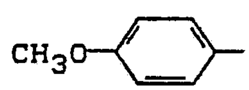 | $CH_3$ | $OCH_3$ | |
| 31 | 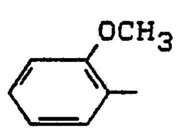 | 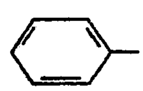 | $CH_3$ | $CH_3$ | 153 |

Tabelle 3 - Fortsetzung

| Beisp.-Nr. | 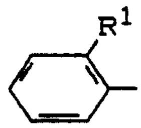 | $R^2$ | $R^3$ | $R^4$ | Fp /[°C] |
|---|---|---|---|---|---|
| 32 | 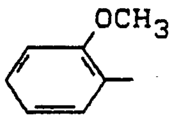 | 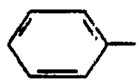 | $CH_3$ | $OCH_3$ | 118 |
| 33 | 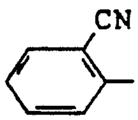 | 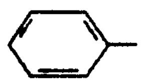 | $CH_3$ | $CH_3$ | |
| 34 | 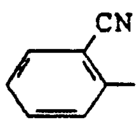 | 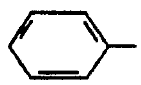 | $CH_3$ | $OCH_3$ | |
| 35 | 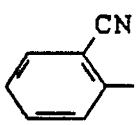 | 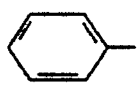 | $OCH_3$ | $OCH_3$ | |
| 36 | 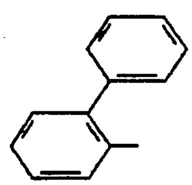 | 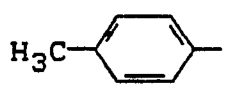 | $OCH_3$ | $OCH_3$ | 139 |
| 37 | 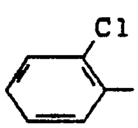 | 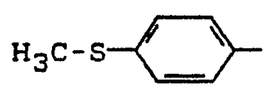 | $OCH_3$ | $OCH_3$ | 124 |
| 38 | 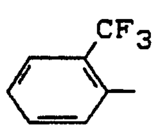 | 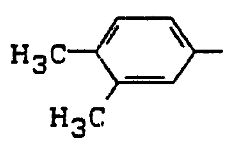 | $CH_3$ | $SCH_3$ | 123 |
| 39 | 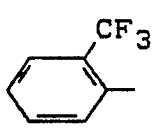 | 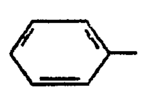 | $CH_3$ | $SCH_3$ | 95 |

Tabelle 3 - Fortsetzung

| Beisp.-Nr. | 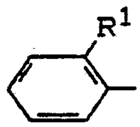 | $R^2$ | $R^3$ | $R^4$ | Fp /[° C] |
|---|---|---|---|---|---|
| 40 | 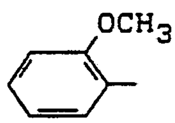 | 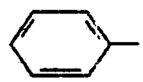 | $CH_3$ | $SCH_3$ | 120 |
| 41 | 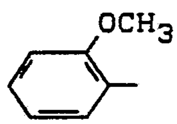 | 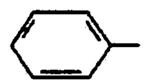 | $CH_3$ | $N(CH_3)_2$ | 151 |
| 42 | 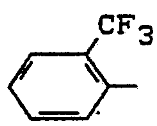 | 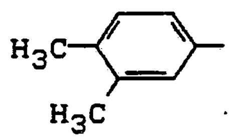 | $OCH_3$ | $OCH_3$ | 128 |
| 43 | 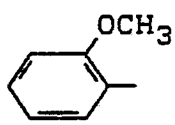 | 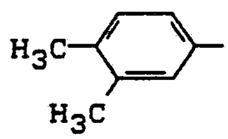 | $OCH_3$ | $OCH_3$ | 123 |
| 44 | 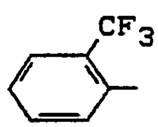 | 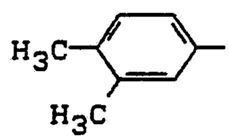 | $CH_3$ | $N(CH_3)_2$ | 143 |
| 45 | 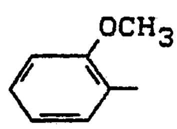 | 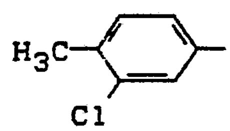 | $CH_3$ | $SCH_3$ | 127 |
| 46 | 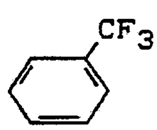 | 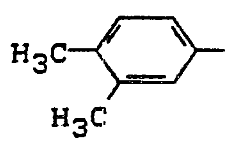 | $CH_3$ | $OCH_3$ | 128 |

Tabelle 3 - Fortsetzung

| Beisp.-Nr. | 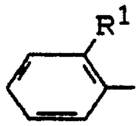 | $R^2$ | $R^3$ | $R^4$ | Fp /[°C] |
|---|---|---|---|---|---|
| 47 | 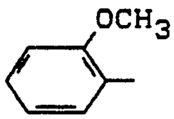 | 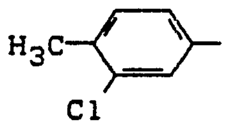 | $CH_3$ | $OCH_3$ | 131 |
| 48 | 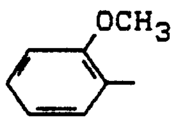 | 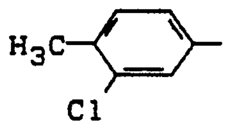 | $CH_3$ | $N(CH_3)_2$ | 147 |
| 49 | 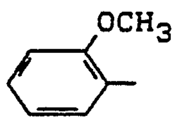 | 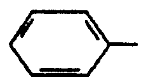 | $CH_3$ | $N(CH_3)_2$ | 162 |
| 50 | 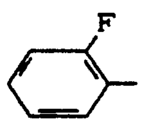 | 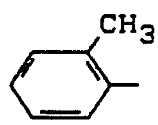 | $CH_3$ | $OCH_3$ | 94 |
| 51 | 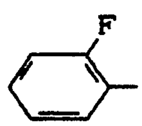 | 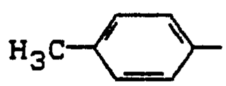 | $CH_3$ | $OCH_3$ | 108 |
| 52 | 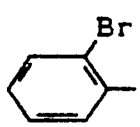 | 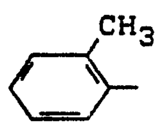 | $CH_3$ | $OCH_3$ | 147 |
| 53 | 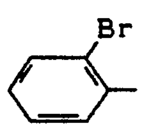 | 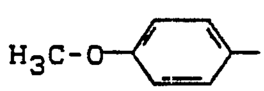 | $CH_3$ | $OCH_3$ | 132 |

Ausgangsverbindungen der Formel (II)

Beispiel (II-1)

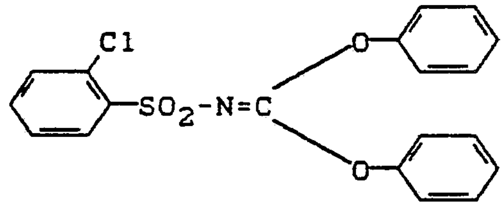

23,2 g (0,085 Mol) 2-Chlorbenzolsulfonylisocyaniddichlorid werden in 100 ml Aceton gelöst und bei 20 °C portionsweise mit 19,7 g (0,085 Mol) Natriumphenolat versetzt. Das Reaktionsgemisch wird 16 Stunden bei 20 °C nachgerührt, über Kieselgur abgesaugt und das Filtrat im Vakuum eingeengt. Der Rückstand wird mit Isopropanol verrieben, abgesaugt, gewaschen und getrocknet.

Man erhält 29,4 g (89 % der Theorie) 2-Chlorbenzolsulfonyliminokohlensäure-O,O-diphenylester vom Schmelzpunkt 130 °C.

Beispiel (II-2)

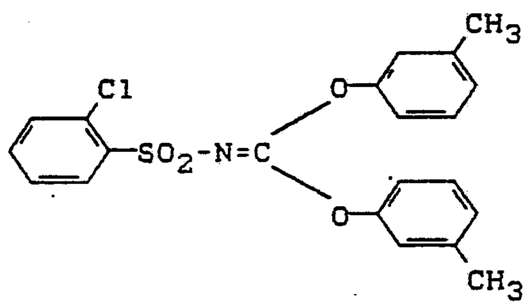

Zu einer Mischung von 27,2 g (0,1 Mol) 2-Chlorbenzolsulfonylisocyaniddichlorid, 21,6 g (0,2 Mol) 3-Methylphenol und 150 ml Toluol werden 22,6 g (0,217 Mol) Triethylamin in der Weise getropft, daß eine Reaktionstemperatur von 40 °C nicht überschritten wird. Das Reaktionsgemisch wird anschließend 3 Stunden bei 25 °C nachgerührt und abgesaugt. Das Filtrat wird mit 200 ml Methylenchlorid extrahiert, die organische Phase mit Wasser gewaschen, über Natriumsulfat getrocknet und eingeengt.

Man erhält 24,8 g (59,7 % der Theorie) 2-Chlorbenzolsulfonyliminokohlensäure-O,O-di-(3-methyl-phenyl)-ester in Form farbloser Kristalle vom Schmelzpunkt 98 °C.

Analog Beispiel (II-1) und (II-2) können die folgenden Verbindungen der Formel (II) hergestellt werden:

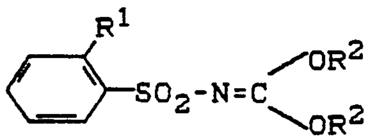 (II)

Tabelle 4

| Beisp.-Nr. | 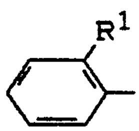 | $R^2$ | Physikal. Konstante |
|---|---|---|---|
| II-3 | 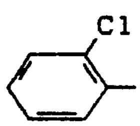 | 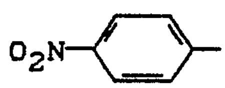 | Fp. 183 °C |

<u>Tabelle 4</u> - Fortsetzung

| Beisp.- Nr. | 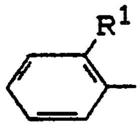 | $R^2$ | Physikal. Konstante |
|---|---|---|---|
| II-4 | 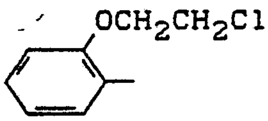 | 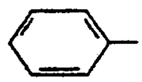 | |
| II-5 | 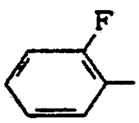 | 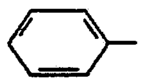 | Fp. 117 °C |
| II-6 | 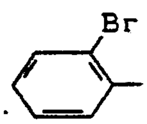 | 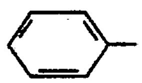 | |
| II-7 | 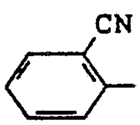 | 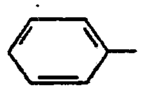 | |
| II-8 | 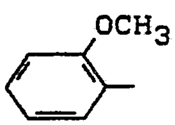 | 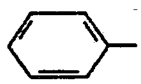 | Fp. 170 °C |
| II-9 | 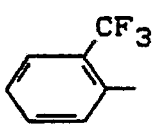 | 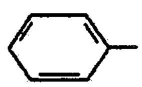 | |
| II-10 | 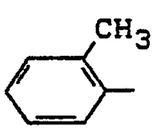 | 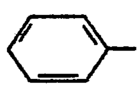 | |
| II-11 | 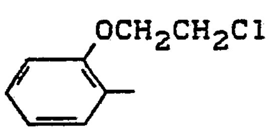 | 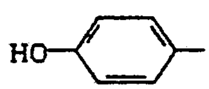 | |

Tabelle 4 - Fortsetzung

| Beisp.-Nr. | 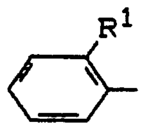 | $R^2$ | Physikal. Konstante |
|---|---|---|---|
| II-12 | 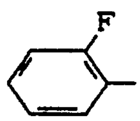 | 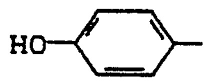 | |
| II-13 | 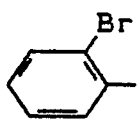 | 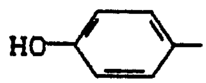 | |
| II-14 | 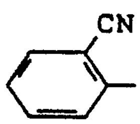 | 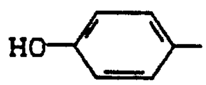 | |
| II-15 | 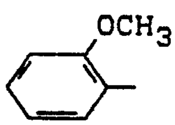 | 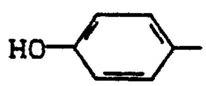 | |
| II-16 | 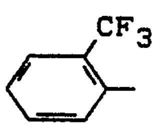 | 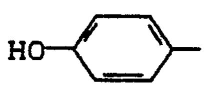 | |
| II-17 | 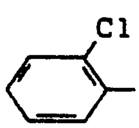 | 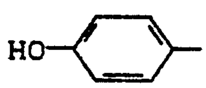 | |
| II-18 | 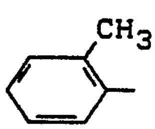 | 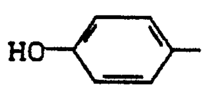 | |
| II-19 | 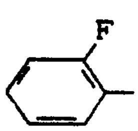 | 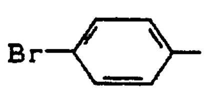 | |

Tabelle 4 - Fortsetzung

| Beisp.-Nr. | 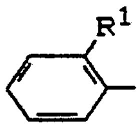 | $R^2$ | Physikal. Konstante |
|---|---|---|---|
| II-20 | 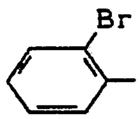 | 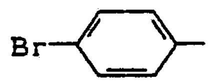 | |
| II-21 | 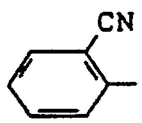 | 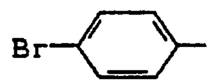 | |
| II-22 | 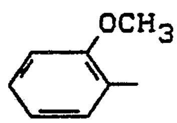 | 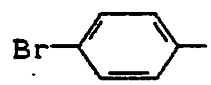 | |
| II-23 | 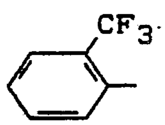 | 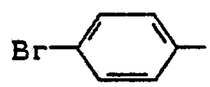 | |
| II-24 | 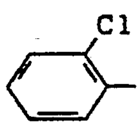 | 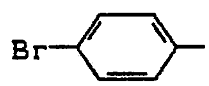 | |
| II-25 | 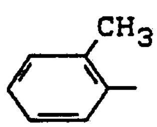 | 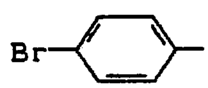 | |
| II-26 | 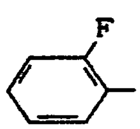 | 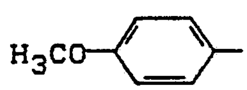 | |
| II-27 | 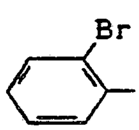 | 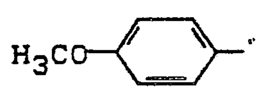 | |

<u>Tabelle 4</u> - Fortsetzung

| Beisp.-Nr. | 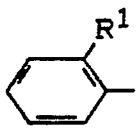 | $R^2$ | Physikal. Konstante |
|---|---|---|---|
| II-28 | 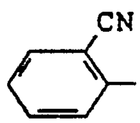 | 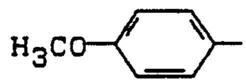 | |
| II-29 | 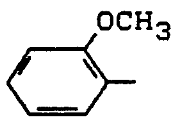 | 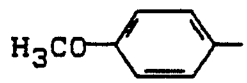 | |
| II-30 | 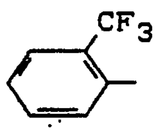 | 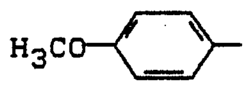 | |
| II-31 | 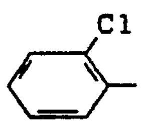 | 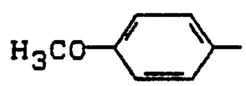 | |
| II-32 | 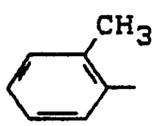 | 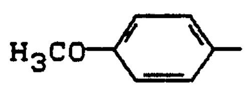 | |
| II-33 | 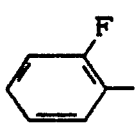 | 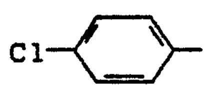 | |
| II-34 | 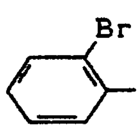 | 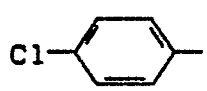 | |
| II-35 | 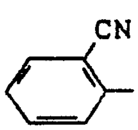 | 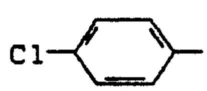 | |

Tabelle 4 - Fortsetzung

| Beisp.-Nr. | 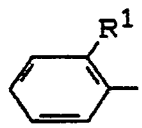 | $R^2$ | Physikal. Konstante |
| --- | --- | --- | --- |
| II-36 | 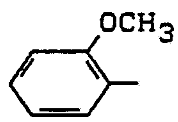 | 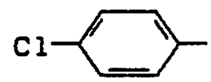 | |
| II-37 | 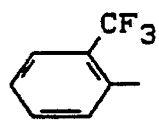 | 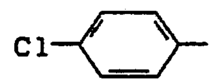 | |
| II-38 | 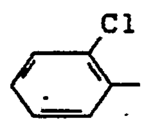 | 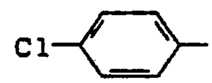 | |
| II-39 | 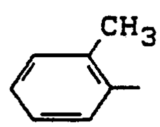 | 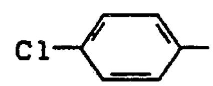 | |
| II-40 | 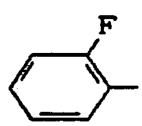 | 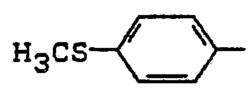 | |
| II-41 | 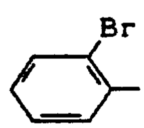 | 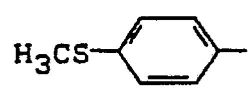 | |
| II-42 | 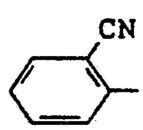 | 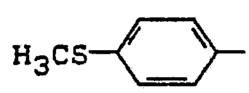 | |
| II-43 | 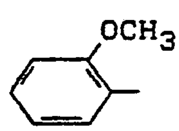 | 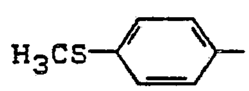 | |

<u>Tabelle 4</u> - Fortsetzung

| Beisp.-Nr. | 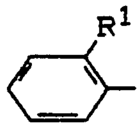 | $R^2$ | Physikal. Konstante |
|---|---|---|---|
| II-44 | 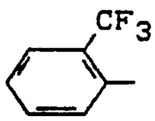 | 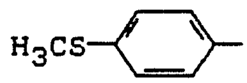 | |
| II-45 | 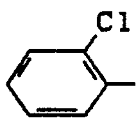 | 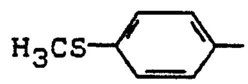 | |
| II-46 | 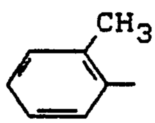 | 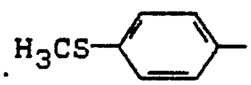 | |
| II-47 | 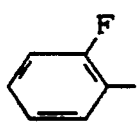 | 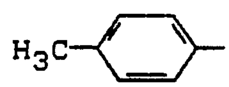 | |
| II-48 | 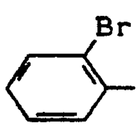 | 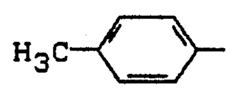 | Fp. 112 °C |
| II-49 | 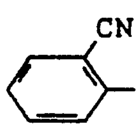 | 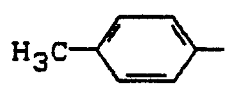 | |
| II-50 | 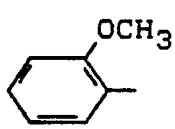 | 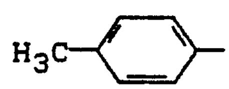 | |
| II-51 | 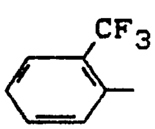 | 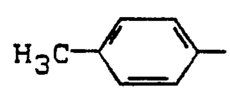 | |

Tabelle 4 - Fortsetzung

| Beisp.-Nr. | 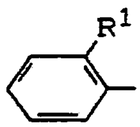 | $R^2$ | Physikal. Konstante |
|---|---|---|---|
| II-52 | 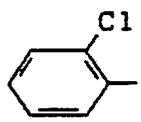 | 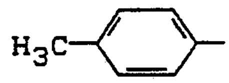 | Fp. 117 °C |
| II-53 | 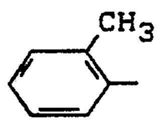 | 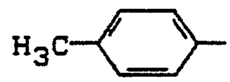 | |
| II-54 | 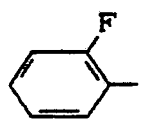 | 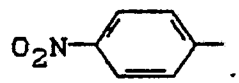 | |
| II-55 | 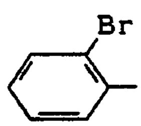 | 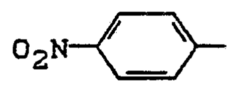 | |
| II-56 | 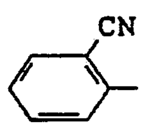 | 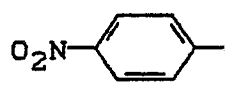 | |
| II-57 | 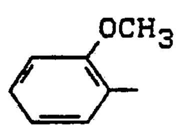 | 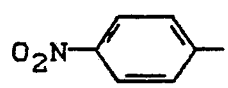 | |
| II-58 | 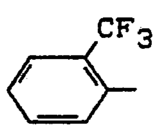 | 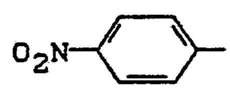 | |
| II-59 | 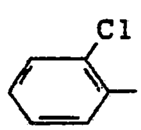 | 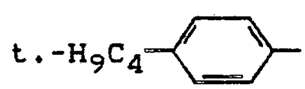 | Wachs |

Tabelle 4 - Fortsetzung

| Beisp.-Nr. | 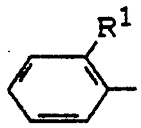 | $R^2$ | Physikal. Konstante |
|---|---|---|---|
| II-60 | 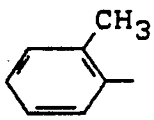 | 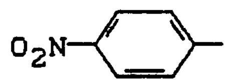 | |
| II-61 | 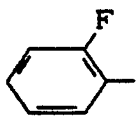 | 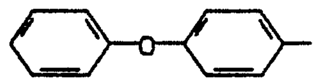 | |
| II-62 | 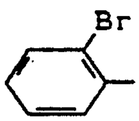 | 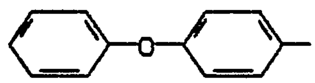 | |
| II-63 | 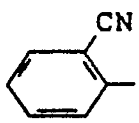 | 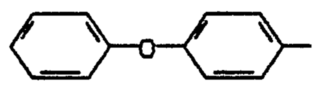 | |
| II-64 | 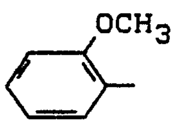 | 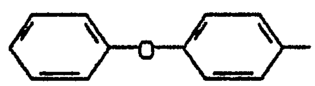 | |
| II-65 | 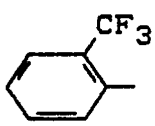 | 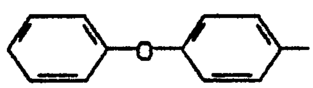 | |
| II-66 | 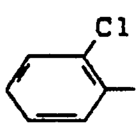 | 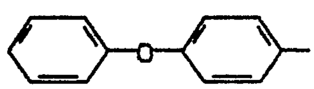 | |
| II-67 | 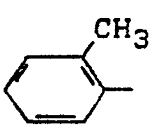 | 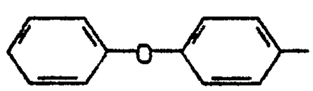 | |

Tabelle 4 - Fortsetzung

| Beisp.-Nr. | 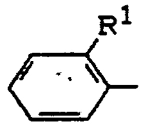 | $R^2$ | Physikal. Konstante |
|---|---|---|---|
| II-68 | 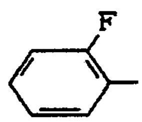 | 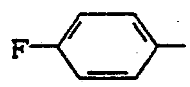 | |
| II-69 | 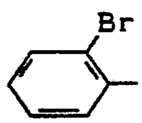 | 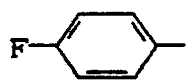 | |
| II-70 | 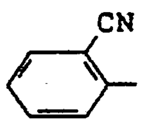 | 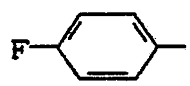 | |
| II-71 | 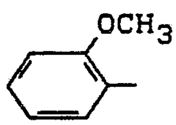 | 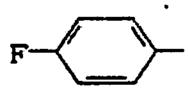 | |
| II-72 | 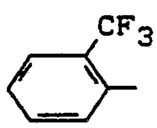 | 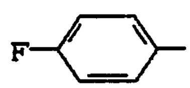 | |
| II-73 | 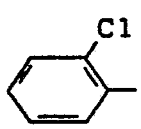 | 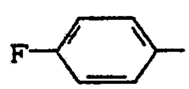 | |
| II-74 | 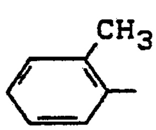 | 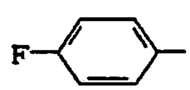 | |
| II-75 | 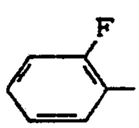 | 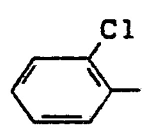 | |

Tabelle 4 - Fortsetzung

| Beisp.-Nr. | 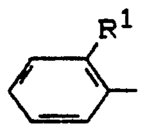 | $R^2$ | Physikal. Konstante |
|---|---|---|---|
| II-76 | 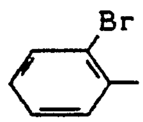 | 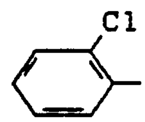 | |
| II-77 | 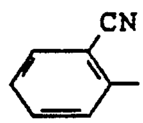 | 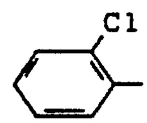 | |
| II-78 | 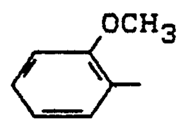 | 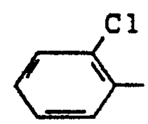 | |
| II-79 | 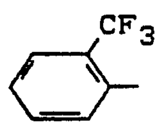 | 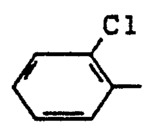 | |
| II-80 | 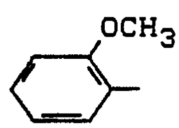 | 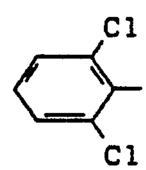 | Fp. 235 °C |
| II-81 | 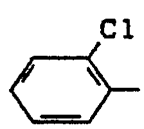 | 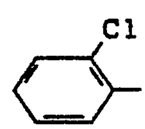 | |
| II-82 | 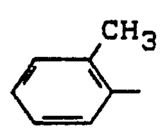 | 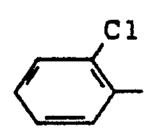 | |

Tabelle 4 - Fortsetzung

| Beisp.-Nr. | 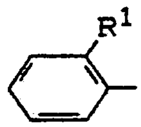 | $R^2$ | Physikal. Konstante |
| --- | --- | --- | --- |
| II-83 | 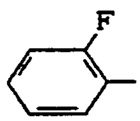 | 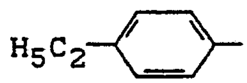 | |
| II-84 | 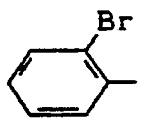 | 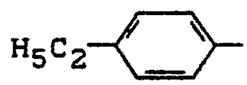 | |
| II-85 | 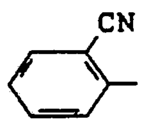 | 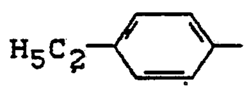 | |
| II-86 | 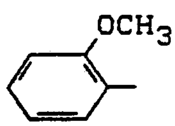 | 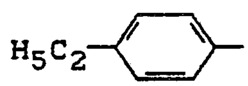 | |
| II-87 | 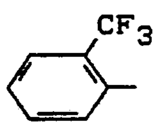 | 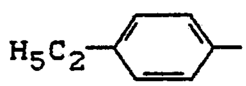 | |
| II-88 | 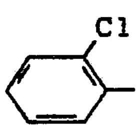 | 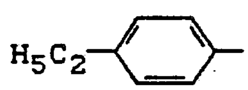 | |
| II-89 | 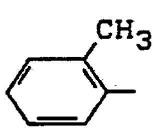 | 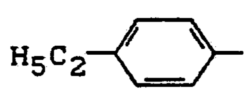 | |
| II-90 | 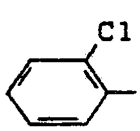 | 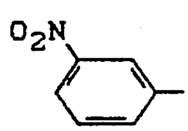 | Fp. 182 °C |

Tabelle 4 - Fortsetzung

| Beisp.-Nr. | 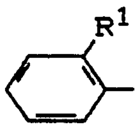 | $R^2$ | Physikal. Konstante |
|---|---|---|---|
| II-91 | 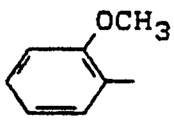 | 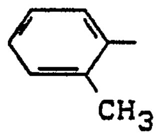 | |
| II-92 | 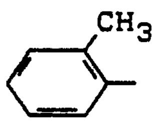 | 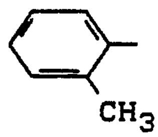 | |
| II-93 | 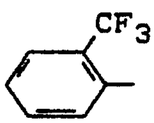 | 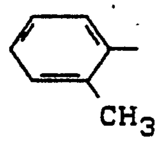 | |
| II-94 | 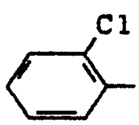 | 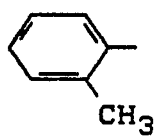 | Fp. 141 °C |
| II-95 | 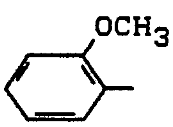 | 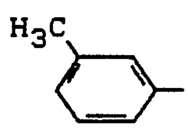 | |
| II-96 | 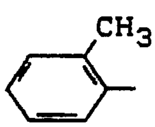 | 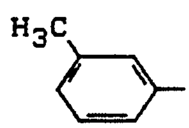 | |
| II-97 | 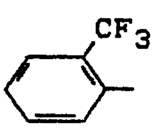 | 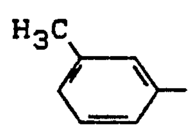 | |

Tabelle 4 - Fortsetzung

| Beisp.-Nr. | 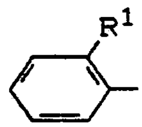 | $R^2$ | Physikal. Konstante |
| --- | --- | --- | --- |
| II-98 | 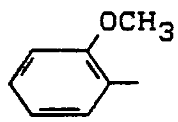 | 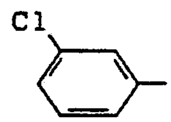 | |
| II-99 | 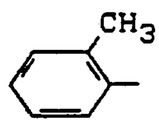 | 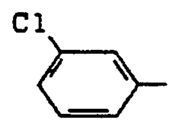 | |
| II-100 | 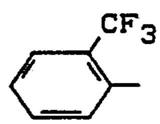 | 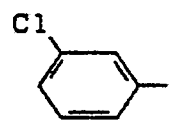 | |
| II-101 | 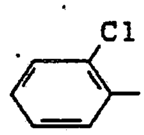 | 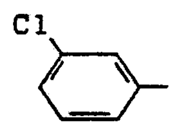 | Fp. 120 °C |
| II-102 | 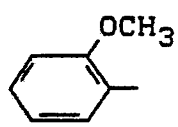 | 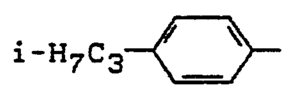 | |
| II-103 | 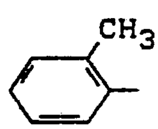 | 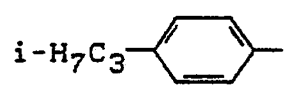 | |
| II-104 | 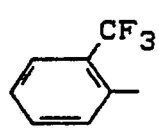 | 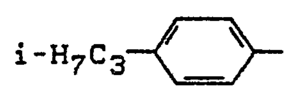 | |
| II-105 | 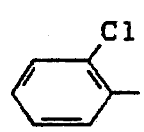 | 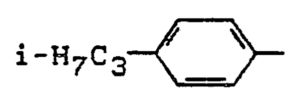 | |

Tabelle 4 - Fortsetzung

| Beisp.-Nr. | 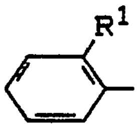 | $R^2$ | Physikal. Konstante |
|---|---|---|---|
| II-106 | 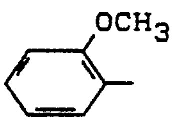 | 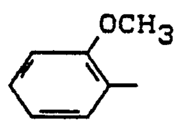 | |
| II-107 | 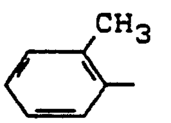 | 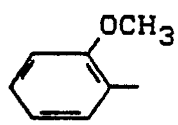 | |
| II-108 | 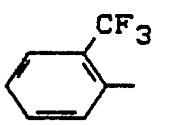 | 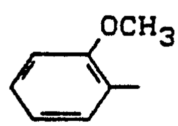 | |
| II-109 | 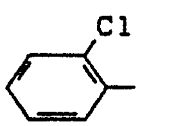 | 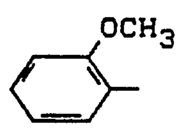 | |
| II-110 | 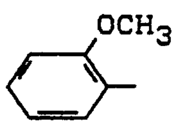 | 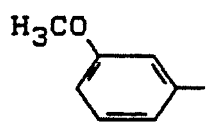 | |
| II-111 | 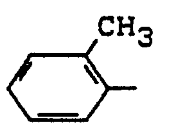 | 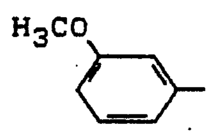 | |
| II-112 | 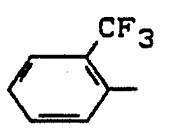 | 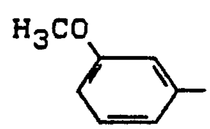 | |
| II-113 | 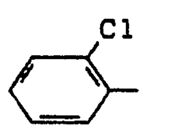 | 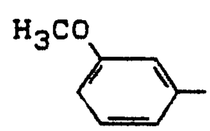 | |

Tabelle 4 - Fortsetzung

| Beisp.-Nr. | 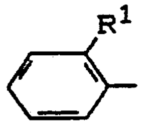 | $R^2$ | Physikal. Konstante |
|---|---|---|---|
| II-114 | 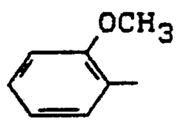 | 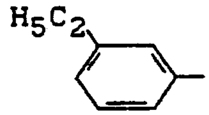 | |
| II-115 | 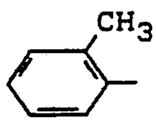 | 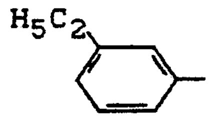 | |
| II-116 | 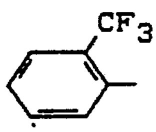 | 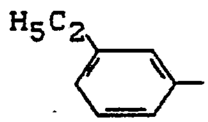 | |
| II-117 | 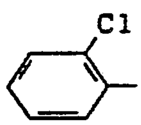 | 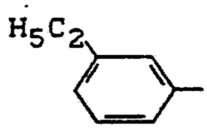 | |
| II-118 | 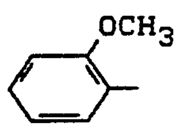 | 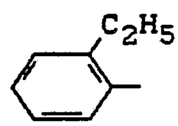 | |
| II-119 | 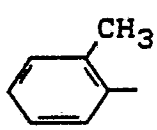 | 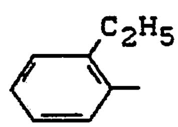 | |
| II-120 | 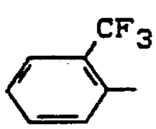 | 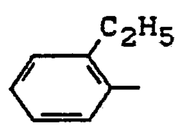 | |
| II-121 | 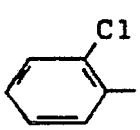 | 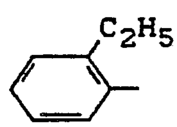 | |

Tabelle 4 - Fortsetzung

| Beisp.-Nr. | 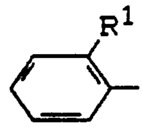 | $R^2$ | Physikal. Konstante |
|---|---|---|---|
| II-122 | 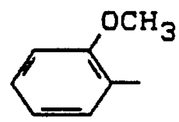 | 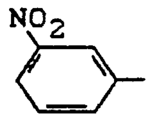 | Fp. 171 °C |
| II-123 | 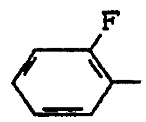 | 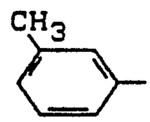 | |
| II-124 | 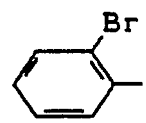 | 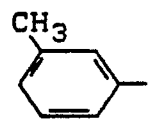 | |
| II-125 | 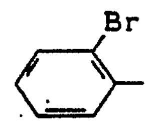 | 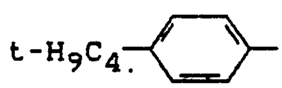 | |

Ausgangsverbindungen der Formel (IV)

Beispiel (IV-1)

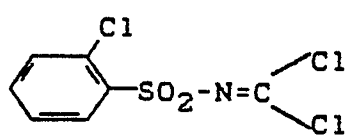

30 g (0,1 Mol) 2-Chlorbenzolsulfonyliminodithiokohlensäure-S,S-dimethylester werden in 200 ml Tetrachlorkohlenstoff gelöst und bei 20 °C werden 71 g (1 Mol) trockenes Chlorgas eingeleitet. Danach wird 30 Minuten nachgerührt und im Vakuum eingeengt. Der Rückstand wird in Petrolether gegossen, das ausgefallene Produkt abgesaugt und getrocknet.

Man erhält 23,3 g (86 % der Theorie) 2-Chlorbenzolsulfonylisocyaniddichlorid vom Schmelzpunkt 74 °C.

Die Herstellung der Verbindung (IV-1) kann auch folgendermaßen durchgeführt werden:

Eine Lösung von 88,7 g (0,3 Mol) 2-Chlorbenzolsulfonyliminodithiokohlensäure-S,S-dimethylester in 200 ml Chloroform wird auf 60 °C erhitzt und bei dieser Temperatur tropfenweise mit 300 g (2,1 Mol) Sulfurylchlorid versetzt. Es wird eine Stunde bei 60 °C nachgerührt bis die Gasentwicklung beendet ist. Anschließend wird eingeengt und der Rückstand andestilliert (Badtemperatur/Druck: 50 °C/200 Pa). Der Rückstand wird mit Petrolether verrieben, abgesaugt und getrocknet.

Man erhält 64 g (78 % der Theorie) 2-Chlorbenzolsulfonylisocyaniddichlorid vom Schmelzpunkt 82 °C.

Analog Beispiel (IV-1) können die nachfolgenden Verbindungen der Formel (IV) hergestellt werden:

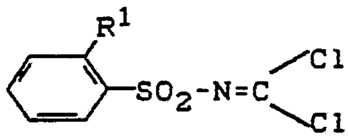 (IV)

<u>Tabelle 5</u>

| Beisp.-Nr. | 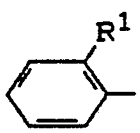 | Phys. Konstante |
|---|---|---|
| IV-2 | 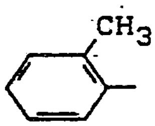 | |
| IV-3 | 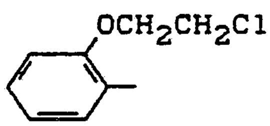 | |
| IV-4 | 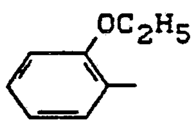 | |
| IV-5 | 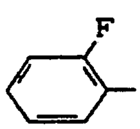 | Kp. 130 °C/600 Pa |

Tabelle 5 - Fortsetzung

| Beisp.-Nr. | 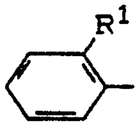 | Phys. Konstante |
|---|---|---|
| IV-6 | 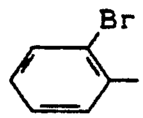 | Fp. 107 °C (Zers.) |
| IV-7 | 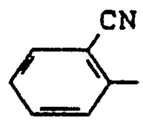 | |
| IV-8 | 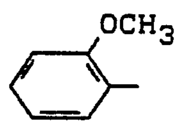 | Öl |
| IV-9 | 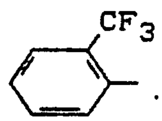 | |

Ausgangsverbindungen der Formel (VI)

Beispiel (VI-1)

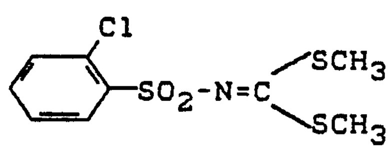

Zu einer Lösung von 20 g (0,1 Mol) 2-Chlor-benzolsulfonsäureamid in 80 ml Dimethylformamid werden bei 20 °C gleichzeitig (aus verschiedenen Tropftrichtern) 8 g (0,2 Mol) Natriumhydroxid - gelöst in 15 ml Wasser - und 6 ml (0,11 Mol) Schwefelkohlenstoff tropfenweise gegeben. Nach einstündigem Rühren werden 13 ml (0,22 Mol) Methylenchlorid zugetropft, und das Reaktonsgemisch wird eine weitere Stunde bei 20 °C gerührt. Durch Zugabe von 500 ml Wasser wird das Produkt ausgefällt und durch Absaugen isoliert.

Man erhält so 22,1 g (75 % der Theorie) 2-Chlor-benzolsulfonyliminodithiokohlensäure-S,S-dimethylester vom Schmelzpunkt 112 °C.

Beispiel (VI-2)

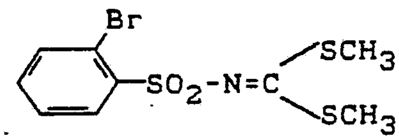

Eine Mischung aus 236 g (1 Mol) 2-Brom-benzolsulfonsäureamid, 1500 ml Dimethylformamid und 85 g (1,11 Mol) Schwefelkohlenstoff wird auf 5 °C abgekühlt und eine Lösung von 80 g (2 Mol) Natriumhydroxid in 150 ml Wasser wird in der Weise zugetropft, daß eine Reaktionstemperatur von 10 °C nicht überschritten wird. Das Reaktionsgemisch wird bei 5 °C - 10 °C noch 30 Minuten nachgerührt, bei dieser Temperatur tropfenweise mit 284 g (2 Mol) Methyliodid versetzt und 18 Stunden bei 20 °C - 25 °C nachgerührt. Die Reaktionsmischung wird in 5 l Wasser gegossen und das ausgefallene Reaktionsprodukt wird abfiltriert. Der Filterrückstand wird in 1,5 l Methylenchlorid aufgenommen und die Methylenchloridphase filtriert, über Natriumsulfat getrocknet und eingeengt. Der Rückstand wird mit 1 l Petrolether verrieben.

Man erhält 290 g (85 % der Theorie) 2-Brom-benzolsulfonyliminodithiokohlensäure-S,S-dimethylester vom Schmelzpunkt 143 °C.

Analog Beispiel (VI-1) und (VI-2) können die in der nachfolgenden Tabelle 6 aufgeführten Verbindungen der Formel (VI) hergestellt werden:

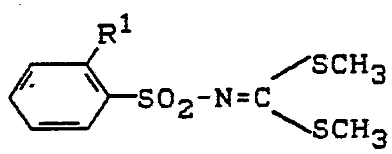

(VI)

Tabelle 6

| Beisp.-Nr. | $R^1$ | Schmelzpunkt / [°C] |
|---|---|---|
| VI-3 | CN | |
| VI-4 | F | 104 |
| VI-5 | $OCH_3$ | 112 |
| VI-6 | $CF_3$ | 124 |

Beispiel A

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

Bei diesem Test zeigen z. B. die Verbindungen der Herstellungsbeispiele (1) und (10) bei der Bekämpfung von dikotylen Unkräutern wie z. B. Datura, Galium, Ipomoea, Sinapis und Viola in Kulturen, wie Gerste und Weizen eine bessere Wirkung als die Vergleichssubstanz (A). (A) = N'-(2-Chlor-benzolsulfonyl)-N"-(4,6-dimethyl-1,3,5-triazin-2-yl)-O-phenyl-isoharnstoff (bekannt aus EP-A 173 957).

Beispiel B

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

Bei diesem Test zeigen z. B. die Verbindungen der Herstellungsbeispiele (1) und (10) bei der Bekämpfung von dikotylen Unkräutern wie Cassia, Galium, Matricaria und Stellaria in Kulturen wie Gerste und Weisen eine bessere Wirkung als die Vergleichssubstanz (A).

## Ansprüche

1) Phenylsulfonylisoharnstoffe der allgemeinen Formel (I)

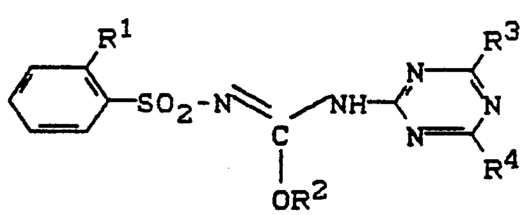

(I)

in welcher

$R^1$ für Halogen, Cyano, $C_1$-$C_6$-Alkyl [welches gegebenenfalls durch Fluor, Chlor, Brom, Cyano, $C_1$-$C_4$-Alkoxy, $C_3$-$C_6$-Cycloalkyl oder Phenyl substituiert ist] oder für $C_1$-$C_4$-Alkoxy [welches gegebenenfalls durch Chlor, Brom, Cyano oder $C_1$-$C_4$-Alkoxy substituiert ist] steht,

$R^2$ für gegebenenfalls substituiertes Aryl steht,

$R^3$ für Wasserstoff, Halogen, Hydroxy, $C_1$-$C_6$-Alkylamino, Di-($C_1$-$C_6$-alkyl)-amino oder für gegebenenfalls substituiertes $C_1$-$C_6$-Alkyl, $C_1$-$C_6$-Alkoxy und $C_1$-$C_6$-Alkylthio steht und

$R^4$ für Wasserstoff, Halogen, Hydroxy, für gegebenenfalls substituiertes $C_1$-$C_6$-Alkyl, $C_1$-$C_6$-Alkoxy und $C_1$-$C_6$-Alkylthio, für $C_1$-$C_6$-Alkylamino oder Di-($C_1$-$C_6$-alkyl)-amino steht,

wobei jedoch die folgende Verbindung ausgenommen wird:

N'-(2-Chlor-benzolsulfonyl)-N"-(4,6-dimethyl-1,3,5-triazin-2-yl)-O-phenyl-isoharnstoff.

2) Phenylsulfonylisoharnstoffe der allgemeinen Formel (I) gemäß Anspruch 1, dadurch gekennzeichnet, daß darin

$R^1$ für Fluor, Chlor, Brom, Cyano, $C_1$-$C_4$-Alkyl [welches gegebenenfalls duch Fluor, Chlor, Brom, Cyano, $C_1$-$C_2$-Alkoxy oder Phenyl substituiert ist] oder für $C_1$-$C_2$-Alkoxy [welches gegebenenfalls durch Chlor, Brom, Cyano oder $C_1$-$C_2$-Alkoxy substituiert ist] steht,

$R^2$ für einen Phenylrest steht, welcher gegebenenfalls substituiert ist durch einen oder mehrere Reste aus der Reihe Halogen, Cyano, Nitro, Hydroxy, Carboxy, $C_1$-$C_6$-Alkyl [welches gegebenenfalls durch Fluor, Chlor, Brom, Nitro, Cyano, Hydroxy, Carboxy, $C_1$-$C_4$-Alkoxy-carbonyl, $C_1$-$C_4$-Alkoxy, $C_1$-$C_4$-Alkylthio oder Phenyl substituiert ist], $C_3$-$C_6$-Cycloalkyl, $C_1$-$C_4$-Alkoxy [welches gegebenenfalls durch Fluor, Chlor, Brom, Cyano, Carboxy, $C_1$-$C_4$-Alkoxy, $C_1$-$C_4$-Alkylthio oder $C_1$-$C_4$-Alkoxy-carbonyl substituiert ist], $C_1$-$C_4$-Alkylthio [welches gegebenenfalls durch Fluor, Chlor, Brom, Cyano, Carboxy, $C_1$-$C_4$-Alkoxy-carbonyl substituiert ist], Amino, $C_1$-$C_4$-Alkyl-amino bzw. Di-($C_1$-$C_4$-alkyl)-amino [welche gegebenenfalls durch Fluor, Chlor, Brom, Cyano, Carboxy, $C_1$-$C_4$-Alkoxy oder $C_1$-$C_4$-Alkoxy-carbonyl substituiert sind], $C_1$-$C_4$-Alkyl-carbonyl-amino, $C_1$-$C_4$-Alkoxy-carbonylamino, (Di)-$C_1$-$C_4$-Alkylamino-carbonyl-amino, Formyl, $C_1$-$C_4$-Alkyl-carbonyl, Benzoyl, $C_1$-$C_4$-Alkoxy-carbonyl, Phenoxy-carbonyl, Benzyloxycarbonyl, Phenyl [welches gegebenenfalls durch Fluor, Chlor, Brom, Cyano, Nitro, Hydroxy oder Methyl substituiert ist], Phenoxy, Phenylthio, Phenylsulfonyl, Phenylamino oder Phenylazo [welche gegebenenfalls durch Fluor, Chlor, Brom, Cyano, Nitro, Methyl und/oder Trifluormethyl substituiert sind], Pyridoxy oder Pyrimidoxy [welche gegebenenfalls durch Fluor, Chlor, Brom, Cyano, Nitro, Methyl und/oder Trifluormethyl substituiert sind], $C_1$-$C_4$-Alkyl-carbonyloxy, $C_1$-$C_4$-Alkoxy-carbonyloxy, $C_1$-$C_4$-Alkyl-amino-carbonyloxy und Di-($C_1$-$C_4$-alkyl)-amino-carbonyloxy, oder welcher gegebenenfalls durch eine Alkylenkette [welche gegebenenfalls verzweigt und/oder durch ein oder mehrere Sauerstoffatome unterbrochen ist] oder einen Benzorest [welcher gegebenenfalls durch Fluor, Chlor, Brom, Cyano, Nitro, Methyl und/oder Trifluormethyl substituiert ist] anelliert ist; worin weiter

$R^3$ für Wasserstoff, Fluor, Chlor, Brom, Hydroxy, $C_1$-$C_4$-Alkylamino, Di-($C_1$-$C_4$-alkyl)-amino, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio [welches gegebenenfalls durch Fluor und/oder Chlor substituiert ist] steht und

$R^4$ für Wasserstoff, Fluor, Chlor, Brom, Hydroxy, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio [welches gegebenenfalls durch Fluor und/oder Chlor substituiert ist], $C_1$-$C_4$-Alkylamino oder Di-($C_1$-$C_4$-alkyl)-amino steht,

wobei jedoch die folgende Verbindung ausgenommen wird:

N'-(2-Chlor-benzolsulfonyl)-N"-(4,6-dimethyl-1,3,5-triazin-2-yl)-O-phenyl-isoharnstoff.

3) Phenylsulfonylisoharnstoffe der allgemeinen Formel (I) gemäß Anspruch 1, dadurch gekennzeichnet, daß darin

$R^1$ für Fluor, Chlor, Brom, Cyano, Methyl, Ethyl, Trifluormethyl, Methoxy, 2-Chlorethoxy oder Benzyl steht,

$R^2$ für einen Phenylrest steht, welcher gegebenenfalls substituiert ist durch einen oder zwei Reste aus der Reihe

Fluor, Chlor, Brom, Iod, Cyano, Nitro, Hydroxy, Carboxy, $C_1$-$C_3$-Alkoxy-carbonyl, $C_1$-$C_4$-Alkyl, Trifluormethyl, Hydroxymethyl, Methoxycarbonylmethyl, Phenyl-$C_1$-$C_3$-alkyl, Cyclohexyl, $C_1$-$C_3$-Alkoxy, Trifluormethoxy, $C_1$-$C_3$-Alkylthio, Trifluormethylthio, Dimethylamino, Amino, Acetylamino, Methylaminocarbonyl, Formyl, Acetyl, Benzoyl, Phenyl, Hydroxyphenyl, Phenoxy [welches gegebenenfalls durch Chlor und/oder Trifluormethyl substituiert ist], Phenylamino, Phenylazo, Pyridoxy [welches gegebenenfalls durch Chlor und/oder Trifluormethyl substituiert ist], oder welcher gegebenenfalls benzanelliert ist; worin weiter

$R^3$ für Fluor, Chlor, Methyl, Ethyl, Methoxy, Ethoxy, Methylthio, Ethylthio, Methylamino, Ethylamino, Dimethylamino oder Diethylamino steht und

$R^4$ für Fluor, Chlor, Methyl, Ethyl, Methoxy, Ethoxy, Methylthio, Ethylthio, Methylamino, Ethylamino, Dimethylamino oder Diethylamino steht,

wobei jedoch die folgende Verbindung ausgenommen wird:

N'-(2-Chlor-benzolsulfonyl)-N"-(4,6-dimethyl-1,3,5-triazin-2-yl)-O-phenyl-isoharnstoff.

4) Verfahren zur Herstellung von Phenylsulfonylisoharnstoffen der allgemeinen Formel (I) gemäß Anspruch 1, dadurch gekennzeichnet, daß man Iminokohlensäureester der Formel (II)

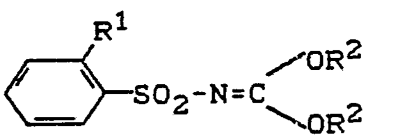

(II)

in welcher
$R^1$ und $R^2$ die in Anspruch 1 angegebenen Bedeutungen haben,
mit 2-Amino-triazinen der Formel (III)

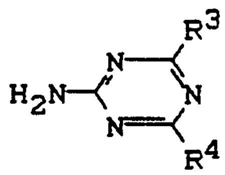

(III)

in welcher
$R^3$ und $R^4$ die in Anspruch 1 angegebenen Bedeutungen haben,
in Gegenwart von Säureakzeptoren und gegebenenfalls in Gegenwart von Verdünnungsmitteln umsetzt.

5) Herbizide Mittel, gekennzeichnet durch einen Gehalt an mindestens einem Phenylsulfonylisoharnstoff der allgemeinen Formel (I) gemäß Anspruch 1.

6) Verwendung von Phenylsulfonylisoharnstoffen der allgemeinen Formel (I) gemäß Anspruch 1 zur Bekämpfung von unerwünschtem Pflanzenwachstum.

7) Verfahren zur Herstellung von herbiziden Mitteln, dadurch gekennzeichnet, daß man Phenylsulfonylisoharnstoffe der allgemeinen Formel (I) gemäß Anspruch 1 mit Streckmitteln und/oder oberflächenaktiven Mitteln vermischt.